(19) Europäisches Patentamt
European Patent Office
Office européen des brevets

(11) Publication number: **0 411 323 A2**

# (12) EUROPEAN PATENT APPLICATION

(21) Application number: **90112469.3**

(22) Date of filing: **29.06.90**

(51) Int. Cl.5: **C12N 15/12**, C12N 15/00, C12N 5/00, G01N 33/68, A01K 67/00

The microorganism(s) has (have) been deposited with Collection Nationale de Cultures de Microorganismes under number(s) I-959 and 963.

(30) Priority: **30.06.89 US 374690**
**29.03.90 US 502140**

(43) Date of publication of application:
**06.02.91 Bulletin 91/06**

(84) Designated Contracting States:
**AT BE CH DE DK ES FR GB GR IT LI LU NL SE**

(71) Applicant: **INSTITUT NATIONAL DE LA SANTE ET DE LA RECHERCHE MEDICALE (INSERM)**
**101, rue de Tolbiac**
**F-75654 Paris Cédex 13(FR)**

Applicant: **LES LABORATOIRES SQUIBB S.A.**
**Tour Generale, 5, Place de la Pyramide, Quartier Villon**
**F-92800 Puteaux(FR)**

Applicant: **L'INSTITUT DE RECHERCHE SQUIBB, G.I.E.**
**Tour Generale, 5, Place de la Pyramide, Quartier Villon**
**F-92800 Puteaux(FR)**

(72) Inventor: **Chambon, Pierre**
**4, rue du Dr. Albert Schweitzer**
**F-67113 Blaesheim(FR)**
Inventor: **Kastner, Philippe**
**28 quai Mullenheim**
**F-67000 Strasbourg(FR)**
Inventor: **Krust, Andrée**
**5, rue Paul Collomp**
**F-67000 Strasbourg(FR)**
Inventor: **Petkovich, Martin**
**875 Everitt Avenue, Kingston, Ontario K7M 4RI(CA)**
Inventor: **Zelent, Arthur**
**1 rue des Lentilles**
**F-67000 Strasbourg(FR)**
Inventor: **Leroy, Pierre**
**6 rue du Faubourg Nation.**
**F-67000 Strasbourg(FR)**
Inventor: **Mendelsoh, Cathy**
**3 rue Kageneck**
**F-67000 Strasbourg(FR)**
Inventor: **Staub, Adrien**
**14 rue des Prés**
**F-67100 Strasbourg(FR)**

(74) Representative: **Josif, Albert et al**
**c/o MODIANO, JOSIF, PISANTY & STAUB, Baaderstrasse 3e 3**
**D-8000 München 5(DE)**

## (54) Human and mouse retinoic acid receptors and genes encoding for them.

(57) The cDNA for mouse and human retinoic acid receptors α, β and γ were cloned, characterized and expressed. The novel human retinoic acid receptor γ is expressed specifically in skin.

EP 0 411 323 A2

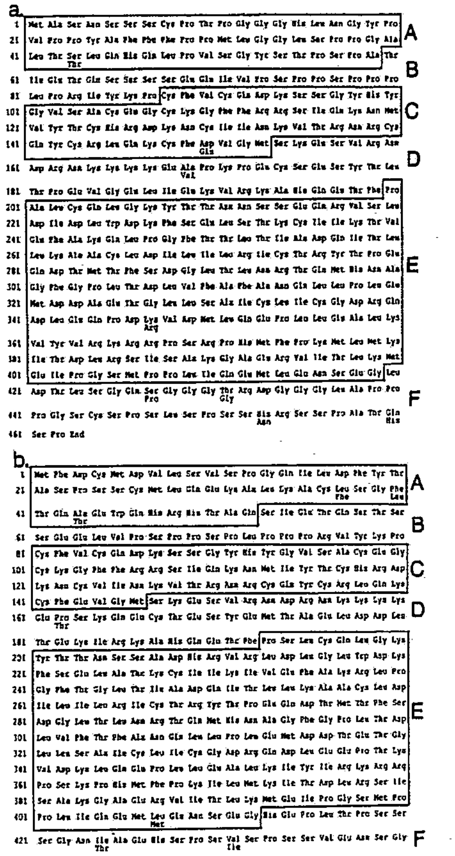

FIG. 1-1

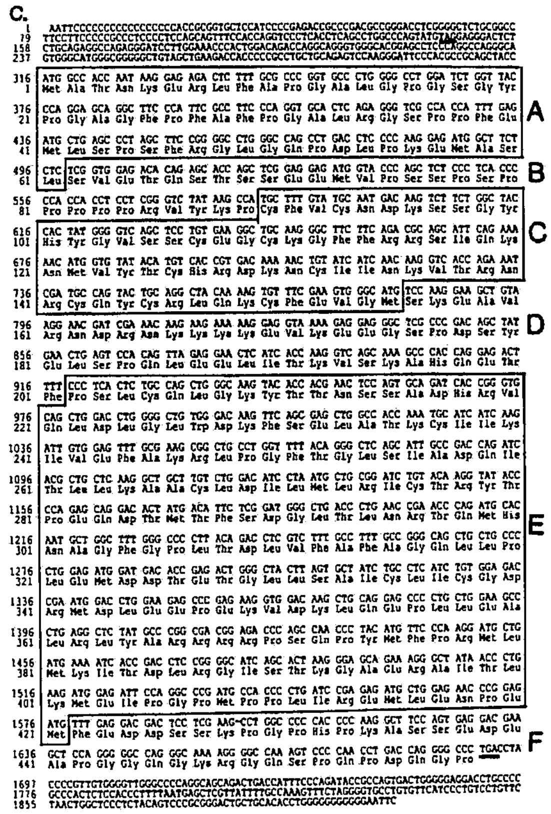

FIG. 1-2

# HUMAN AND MOUSE RETINOIC ACID RECEPTORS AND GENES ENCODING FOR THEM

The present invention relates to human retinoic acid receptor γ (hRAR-γ) and mouse retinoic acid receptors α, β and γ (mRAR-α, mRAR-β and mRAR-γ) and genes encoding them.

A mouse RAR-γ RNA was found to be associated almost exclusively and at comparatively high levels in skin. A cDNA for the human counterpart of the mRAR-γ was cloned and characterized. The corresponding mRNA was found to be the predominant retinoic acid receptor (RAR) RNA species in skin.

The present invention is based on the discovery of cDNA's, coding for mouse retinoic acid receptors α, β and γ and human retinoic acid receptor γ. The respective cDNA's were cloned, inserted into eukaryotic expression vectors and expressed in cells. Mouse retinoic acid receptors α, β and γ and human retinoic acid receptor γ were produced and shown to trans-activate transcription in a retinoic acid (RA)-dependent manner. RAR-γ was found to be specifically expressed in skin.

The present invention also concerns the discovery of cDNA's coding for isoforms of mouse retinoic acid receptor γ. These isoforms are conserved from mouse to human and exhibit a specific pattern of expression.

In the present specification and claims, reference will be made to phrases and terms of art which are expressly defined for use herein as follows:

Nuclear receptor multigene family : a multigene family of proteins which act as inducible enhancer factors.

cDNA : DNA complimentary to mRNA.

*trans*-activation: activation of initiation of transcription by a protein factor exogenous to the DNA and therefore acting in *trans*.

chimaeric receptor : an engineered receptor made up of domains originating from different receptors.

reporter plasmid : a plasmid containing a reporter gene.

reporter gene : a gene whose promoter is under the control of the ligand-responsive element of a given nuclear receptor.

ligand : a molecule which interacts specifically with a given nuclear receptor and activates it.

inducible enhancer factor : a trans-acting protein which, upon binding of a cognate ligand, activates initiation of transcription by binding to a cognate DNA responsive element (enhancer).

promoter : the flanking region of a gene which controls initiation of transcription.

transfection : introduction of a recombinant plasmid into a cell in culture.

retinoic acid receptor : a member of the nuclear receptor family which binds RA specifically.

responsive element : the DNA *cis-element (= enhancer) which mediates the trans*-activation by a cognate nuclear receptor.

consensus sequence : the minimal sequence common to all responsive elements mediating the effect of a given nuclear receptor.

ER : estrogen receptor.

PR : progesterone receptor.

GR : glucocorticoid receptor.

RAR : retinoic acid receptor.

vit-tk-CAT : an estrogen responsive reporter gene.

MMTV-CAT : glucocorticoid responsive reporter gene.

RARO : a recombinant RAR expression vector.

RA : retionic acid.

CRABP : cellular retinoic acid binding proteins.

CRBP : cellular retinol binding proteins.

hRAR-α, β and γ: human retinoic acid receptors α, β and γ.

mRAR-α, β and γ: mouse retinoic acid receptors α, β and γ.

ORF : open reading frame.

Isoform : alternative mRNA or protein generated from a given gene.

5′-UTR : 5′-untranslated region.

PCR : polymerase chain reaction.

EC cell : embryonal carcinoma cell.

ES cell : embryonic stem cell.

kb : kilobase.

Three RARs were found to be expressed in the mouse, two of them being highly homologous to the RAR-α and β previously characterized in human and a third mRAR-γ which is associated with skin. It was observed that hRAR-α and mRAR-α, and hRAR-β and mRAR-β are more homologous to each other than either hRAR-α and hRAR-β, or mRAR-α and mRAR-β, which strongly suggests that the three members of the RAR subfamily exert specific functions during embryogenesis, differentiation and in adult tissues, perhaps by regulating the transcription of different genes at different times of development and in different tissues. In this respect, it is noteworthy that mRAR-γ expression appears to be highly restricted to skin which is known to require RA for normal development and to be very sensitive to RA treatment in both normal and pathological states [Lotan, R. Biochim. Biophys. Acta 605 , 33-91 (1980); Roberts, A. B. & Sporn, M. G. in the Retinoids vol. 2 (eds Sporn, M. B., Roberts, A. B. & Goodman, D. S.) 209-286 (Academic Press Orlando, Florida, 1984); Fuchs, E. Trends Genet. 4 , 277-281 (1988); Dhouailly, D., Hardy, M. H. & Sengel, P. J. Embryol. exp. Morph. 58 , 63-78 (1980); Peck, G. L. in The Retinoids vol.

2. (eds Sporn, M. B., Roberts, A. B. & Goodman, D. S.) 391-411 (Academic Press Orlando, Florida 1984].

mRAR-γ cDNA was then used to clone its human counterpart (hRAR-γ) from a T47D breast cancer cell cDNA library. Using a transient transfection assay in HeLa cells and a reporter gene harbouring a synthetic RA responsive element, it was demonstrated that hRAR-γ cDNA indeed encodes a RA-inducible transcriptional trans-activator. hRAR-γ RNA is the predominant RAR RNA species in human skin which suggests that hRAR-γ mediates some of the retinoid effects in this tissue.

The mRAR-γ cDNA clone was also used to clone 7 different murine retinoic acid receptor γ cDNA isoforms (designated mRAR-γ1 to γ7), which are generated by alternative splicing of at least 7 exons. These isoforms are different from each other in their 5′-UTR, and in two cases (mRAR-γ1 and γ2) differ in their N-terminal A region, which is known to be important for differential trans-activation by other nuclear receptors.

## The Mouse System

### Cloning of mRAR-α, mRAR-β and mRAR-γ

An 11.5 day-old total mouse embryo λgt10 cDNA library was screened with [$^{32}$P]-labelled hRAR-α and β cDNA probes. 81 clones in total were isolated, mapped by restriction analysis and Southern blotting, and selected clones were sequenced. Two sets of clones were identified as mRAR-α and mRAR-β on the basis of a 98% homology of their cDNA-deduced amino acid sequence with that of hRAR-α and β, respectively (Fig. 1a and b). For both proteins there are only 8 amino acid substitutions (mostly conservative) between the human and mouse sequences. A lesser degree of homology with either RAR-α or β was found for a third set of clones, although the deduced amino acid sequence (Fig. 1c) was obviously related to both of them. This new member of the mouse RAR subfamily was designated mRAR-γ.

In Figure 1 nucleotides and amino acids are numbered on the left side of each sequence. An in frame termination codon (positions 145-147) in the 5′-untranslated region of mRAR-γ cDNA is underlined in (c). Regions A, C and E are boxed, and the location of regions A-F is indicated on the right side of each sequence. The amino acids which differ between mRAR-α and β and their human cognates are indicated in (a) and (b) under the murine sequences. The termination codon at the end of the mRARγ sequence is underlined in (c) and the ends of mRARα and β amino acid sequences are indicated in (a) and (b).

The mRAR-γ sequence contains a major open reading frame which encodes a 458 amino acid protein (Mr 50,897). Alignment of the mRAR-γ sequence with those of mRAR-α and β revealed extensive amino acid sequence similarities as shown in Fig. 2. In Fig. 2, the single letter amino acid code is used, and the number of the last amino acid in each sequence is given at the end of the alignment. Numbers on the left side correspond to the first amino acid in a given line. Regions A, C and E are boxed and regions A-F are designated with capital letters on the right side of the Figure. The non-conserved part within region D is boxed with a hatched line. Gaps have been introduced to obtain the optimal alignment of the mRAR-γ sequence with that of mRAR-α and mRARβ. Dashes represent mRAR-α and β amino acids which are identical with those of mRAR-γ.

The A-F regions indicated in Figs. 1 and 2 were as previously defined by sequence comparison between all members of the nuclear receptor family and hRAR-α and β (Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988); and Green, S. & Chambon, P. Trends Genet. 4 . 309-314 (1988) and references therein). The highest amino acid sequence similarities between mRARs were found in the regions corresponding to the DNA binding domain (region C, 95% amino acid identity), and the ligand binding domain (region E, 85% identity between mRAR-α and mRAR-γ, and 90% identity between mRAR-β and either mRAR-α or γ) of the nuclear receptor family, suggesting that mRAR-γ recognizes the same responsive element and binds the same ligand as mRAR-α and β (see below). Region B was also well conserved showing 75%, 86% and 79% amino acid identity between mRAR-γ and α, mRAR-γ and β, and mRAR-α and β, respectively, whereas no conservation was seen in this region when comparing nuclear receptors which bind different ligands [Green, S. & Chambon, P. Trends Genet. 4 , 309-314 (1988) and references therein]. The D region, which within a given species is also not conserved across the nuclear receptor family and may act as a hinge region [Green, S. & Chambon, P. Trends Genet. 4 , 309-314 (1988)], is less conserved overall between mRARS. However, both the N and C terminal segments of region D are highly conserved, whereas the central segment is not (hatched box in Fig. 2). Finally no significant sequence homology was found between mRARs in region A encoded in an exon different from that encoding region B, ( See , Brand, N., Petkovich, M., Krust, A., Chambon, P., de The, H., Marchio, A., Tiollais, P. & Dejean, A. Nature 332 , 850-853 (1988)], nor in region F, which is also not conserved within a given species between the different nuclear receptors [Green, S. & Chambon, P. Trends Genet. 4 , 309-314 (1988)].

In contrast, there is an almost complete amino acid sequence conservation between the A regions of hRAR-α [Petkovich, M., Brand, N. J., Krust, A. & Chambon, P. Nature 330 , 444-450 (1987); Giguere, V., Ong, E. S., Segui, P. & Evans, R. M. Nature 330 , 624-629 (1987)] and mRAR-α (98%), and hRAR-β [Brand, N., Petkovich, M., Krust, A., Chambon, P., de The, H., Marchio, A., Tiollais, P. & Dejean, A. Nature 332 , 850-853 (1988); Benbrook, D., Lernhardt, E. & Pfahl, M. Nature 333 , 669-672 (1988)] and mRAR-β (94%), as well as between the corresponding F regions (90% for hRAR-α and mRAR-α, and 92% for hRAR-β and mRAR-β). Similarly, the entire D region of a given RAR member appears to be conserved across species (98% amino acid identity between both hRAR-α and mRAR-α, and hRAR-β and mRAR-β). This high degree of conservation of the A, D and F regions for a given member of the RAR subfamily contrasts with the lack of, or lower conservation of the same regions between the various RARs within a given species (see above) and also with the lower conservation of regions A/B, D and F for a given steroid hormone receptor across species [Green, S. & Chambon, P. Trends Genet. 4 309-314 (1988); White, R., Lees, J. A., Needham, M., Ham, J. & Parker, M. Molec. Endocrin. 1 , 735-744 (1987) and references therein]. This strongly suggests that the A, B, D and F regions may have specific functions not performed by the C and E regions, but necessary for the three RARs to exert their specific physiological roles. In this respect it is noted that a similar interspecies conservation is seen for the A/B and D regions of the two members of the thyroid hormone receptor subfamily [Murray, M. B., Zilz, N. D., McCreary, N. L., MacDonald, M. J. & Towle, H. C. J. Biol. Chem. 263 , 12770-1277 (1988); Izumo, S. & Mahdavi, V. Nature 334 , 539-542 (1988); Nakai, A., Seino, S., Sakurai, A., Szilak, I., Bell, G. I. & DeGroot, L. J. Proc. Natl. Acad. Sci. USA 85 , 2781-2785 (1988); Weinberger, C., Thompson, C. C., Ong, E. S., Lebo, R., Gruol, D. J. & Evans, R. M. Nature 324 , 641-646] and that region A of the oestrogen and progesterone receptors has been implicated in specific transcriptional trans-activation of some target genes [Green, S. & Chambon, P. Trends Genet. 4 , 309-314 (1988); Tora, L., Gronemeyer, H. Turcotte, B., Gaub, M.P. & Chambon, P. Nature 333 , 185-188 (1988) and references therein].

## Demonstration of Function of mRAR cDNAs

To demonstrate that mRAR-α, β and γ cDNAs encode functional RARs, they were inserted into the eukaryotic expression vector pSG5 [Green, S., Issemann, I. & Scheer, E. Nucleic Acids Res. 16 , 369 (1988)] (mRAR-αO, mRAR-βO and mRAR-γO, respectively) and co-transfected into HeLa cells with a reporter plasmid, $(TRE_3)_3$-tk-CAT, containing a RA responsive element. A clear RA-dependent transactivation was seen in the presence of all-trans RA as seen in Fig. 3. In Fig. 3 HeLa cells were co-transfected as described in example 2 [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)] with pSG5-based expression vectors [Green, S., Issemann, I. & Scheer, E. Nucleic Acids Res. 16 , 369 (1988)] containing the entire cDNA of either mRAR-α (mRAR-αO), β (mRAR-βO) or γ (mRAR-γO), and a reporter plasmid, $(TRE_3)_3$-tk-CAT. carrying a synthetic RA responsive element (RARE). 24 hours after transfection, cells were fed with media containing increasing concentrations of RA as indicated (from 0 to $1x10^{-6}$ M, lanes 1-8, respectively) and harvested 48 hours post-transfection for determination of CAT activity. All three receptors responded apparently similarly to increasing concentrations of RA and maximum activity was achieved at approximately $10^{-7}$M (as previously reported for hRAR-α and β [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)], retinol was much less efficient at the same concentrations - data not shown). No obvious difference was observed between the dose responsiveness of mRAR-α and β, in contrast with previous results obtained with human chimeric constructs in which the C region of hRAR-α and β was replaced by the corresponding region of the human oestrogen receptor [Brand, N., Petkovich, M., Krust, A., Chambon, P., de The, H., Marchio, A., Tiollais, P. & Dejean, A. Nature 332 , 850-853 (1988)]. The reason for these differences is unknown, but may be related to the use of a different and less sensitive responsive element in the present study. Direct determination of the true affinity of RAR-α, β and γ for RA must await direct binding measurements *in vitro* in the absence of the cellular retinoic acid binding protein.

## Expression of mRAR in Mouse Tissues

The expression of mRARs in mouse tissues was investigated by Northern blot analysis of polyA+ RNA using [$^{32}$P]-labelled probes corresponding to either specific oligonucleotides (mRAR-α and mRAR-γ) or randomly primed cDNAs (mRAR-β and mRAR-γ) under stringent conditions. Fig. 4 shows northern blot analysis of mRAR-α, β and γ poly(A)+ RNA from various mouse tissues (as indicated in a, lanes 1-14), and F9 teratocarcinoma cells (b, lanes 15-17) before (lane 15), and after 24 hours (lane 16) and 48 hours (lane 17) treatment with RA ($3.3x10^{-7}$). The position of 28S and 18S rRNAs on the blots is indicated throughout each panel. 4 µg of poly(A)+ RNA was loaded in all lanes. In estimating the molecular sizes of the mRAR's RNAs the 28S and 18S rRNA standards were taken as 4712 [Hassouna, N., Michot, B. & Bachellerie, J. P. Nucleic Acids Res. 12 , 3563-3583 (1984)] and 1869 [Raynal, F., Michot, B. & Bachellerie, J. P. FEBS Letters 167 , 263-268 (1984)] bases-long, respectively. The method is according to Example 3. In Fig. 4 (a), mRAR-α and γ sequences were detected with specific [$^{32}$P]-end labelled oligonucleotide probes derived from region A of each receptor (spanning amino acids 4 through 17 for mRAR-α and 7 through 20 for mRAR-γ, see Fig. 1a and c, respectively). The entire mRAR-β cDNA labelled with [$^{32}$P] by random priming was used as a probe to detect mRAR-β RNA. All hybridizations were performed using the same set of blots, which were first hybridized with the mRAR-α specific probe. After 7 days of exposure at -80° with two intensifying screens and Kodak XRO-5 film, the blots were dehybridized and hybridized with the mRAR-β cDNA probe. The same steps were repeated to detect mRAR-γ RNA. The exposure time in the latter two cases was 4 days at -80°C with two intensifying screens. The lower panel corresponds to a different blot, which was only hybridized with a randomly primed entire mRAR-γ cDNA probe in order to enhance the detection of mRAR-γ sequences in poly(A)+ RNAs from various tissues (the exposure time was 24 hours at -80°C with two intensifying screens). When hybridized with a Syrian hamster cytoskeletal actin cDNA probe [Dodemont, H. J., Soriano, P., Quax, W. J., Ramaekers, F., Lenstra, J. A., Groenen, M. A. M., Bernardi, G. & Bloemedal, H. EMBO J. 1 , 167-171 (1982)], actin RNA was revealed in all cases except for pancreas and adrenal preparations suggesting RNA degradation in these two cases (data not shown). Two mRAR-α specific transcripts (apparent lengths, 3.8 kb and 2.8 kb) were found in RNA prepared from all tissues including skin, and from 11.5 day whole embryo (no conclusion could be drawn for pancreas and adrenal). mRAR-β RNA was detected as a major band of approximately 3.4 kb. When compared with mRAR-α RNA, it was relatively higher in brain and in total 11.5 day embryo, and lower in skin and lung than in other tissues. No. 3.4 kb mRAR-β RNA could be detected in spleen, intestine and testis, and an additional 1.9 kb hybridizing species was revealed only in muscle. Note that due to the use of different probes and exposure conditions for the autoradiograms, the mRAR-β signal was amplified by at least 20-fold relative to the mRAR-α signal in Fig. 4a. In contrast to the mRAR-α and β RNA patterns, it is remarkable that mRAR-γ RNA was detected at levels at least as high as those of mRAR-α only in the skin of both 4 day-old and adult animals (compare the upper and third row in Fig. 4a for which oligonucleotide probes were used). Using a cDNA probe, mRAR-γ RNA could be additionally revealed in total 11.5 day embryo and in lung, and at trace level in spleen (lower row in Fig. 4a). In Fig. 4b, three independent blots were hybridized with randomly primed [$^{32}$P]-labelled entire mRAR-α, β and γ cDNA probes. Filters were exposed for 12 hours at -80°C using two intensifying screens and Kodak XRO-5 film. After exposure, the filters were dehybridized and probed with [$^{32}$P]-labelled actin cDNA probe (lower panel, ACT).

F9 teratocarcinoma stem cells differentiate to endodermal-like cells upon exposure to RA [Strickland, S. & Mahdavi, V. Cell 15 , 393-403 (1978)]. Using cDNA probes, mRAR-α, and γ RNAs were readily detected in these cells under conditions where the mRAR-β RNA signal could not be seen (Fig. 4b). Treatment with RA for 24 and 48 hours led to an induction of mRAR-β RNA (at least 30-fold as judged from densitometric scanning), whereas no significant variation was seen for mRAR-α RNA, and mRAR-γ RNA decreased by 2-fold. A similar increase of mRAR-β RNA following RA treatment of F9 cells was observed by L. Gudas and her collaborators (personal communication). Whether this induction occurred at the transcriptional level, as shown recently for hRAR-β in a human cell line [de The, H., Marchio, A., Tiollais, P. and Dejean, A. EMBO J. 8 , 429-433 (1989)] remains to be established. Since both mRAR-α and mRAR-γ are present in undifferentiated F9 cells, it will be interesting to determine whether only one or both of these RARs is responsible for the increase in mRAR-β RNA.

## The Human System

### Cloning of human RAR-γcDNA

When Northern blots of poly(A)+ RNA prepared from the human T47D breast cancer cell line were hybridized with a [$^{32}$P]-labelled cDNA probe corresponding to the entire open reading frame (ORF) of mouse RAR-γ (mRNA-γ), as described in Example 4 and 5, a 3.3 kb cross-hybridizing species could be detected which was distinct from those observed using either hRAR-α or hRARβ - (see below and data not shown). Randomly primed T47D cell cDNA libraries constructed in λgt11 and λgt10 and [$^{32}$P]-labelled mRAR-γ cDNA were used to isolate the corresponding cDNA clones. Positive clones were further processed for sequence analysis. Two of these clones, hRAR-γA and hRAR-γD (2.0 and 1.5 kb in length, respectively) contained a common major ORF which conceptually encodes a 454 amino acid-long protein (Mr 50,347), exhibiting a 97% homology with mouse mRAR-γ (458 amino acid-long).

Fig. 5 shows the nucleotide sequence of the hRAR-γA clone from the first nucleotide following the EcoRI linker to nucleotide 1894. The amino acid sequence representing the ORF common to most of the clones analyzed is shown underneath their respective codons from the assigned initiation ATG codon. The numbers on the left refer to the position of the nucleotides and on the right to those of the amino acids. The sequence was divided into six regions (A-F). Regions A, C and E are boxed. Boxed amino acid residues represent those encoded by the cDNA sequence for mRAR-γ which differ from those encoded by the hRAR-γ cDNA. Half of these differing amino acids are located at the C-terminal end of the proteins and result from a shift in the codon reading frame arising from a single thymidilic residue insertion at position 1763 of hRAR-γA (Fig. 5). This frame- shift was also found in hRAR-γD and hRAR-γE cDNA clones (see below). In contrast to mRAR-γ, there is no in-frame stop codon upstream of the designated methionine initiation codons of hRAR-γA (see Fig. 5) and hRAR-γD (see below, Fig. 6). This assigned position for the initiating codon for hRAR-γA and D, is therefore only tentative and based on the overall high homology of hRAR-γ and mRAR-γ amino acid sequences (see above). It is important to note that the nucleotide sequence of hRAR-γA upstream from the initiating codon of the common ORF (positions 130-414) is 77% homologous to the corresponding 5′ untranslated region of mRAR-γ cDNA.

## Multiple forms of hRAR-γ cDNA differing in their 5′ regions

Fig. 6 shows an alignment of the 5′ sequences of 5 divergent human RAR-γ cDNA clones (indicated at the right hand side of the Figure as hRAR-γ A through E). For each cDNA the numbers on the left refer to the position of the first nucleotide in each line with respect to the most 5′nucleotide (numbered from 1 in each case). The size of each of the hRAR-γ cDNA's is indicated following the most 3′-nucleotide. Gaps in homology are shown by dots. The nucleotides which in hRAR-γC, D, and E cDNAs are divergent from those of hRAR-γA are underlined. The arrowhead (∇) indicates the position separating regions A and B. The assigned initiation ATG for the hRAR-γ ORF common to hRAR-γA, C and D, and the upstream stop codon in frame with this ATG in hRAR-γC are boxed, as well as the upstream in frame ATG in hRAR-γD. Although hRAR-γA and D cDNAs encode a common 454 amino acid-long sequence, they diverge for the first 20 bases at the 5′ end of hRAR-γD cDNA (Fig. 6, underlined sequence). Note that these divergent bases would place in-frame an upstream methionine codon (boxed in Fig. 6) conceptually adding 13 amino acid residues to the common 454 amino acid sequence. Three additional hRAR-γ cDNA clones were found which are not colinear with either hRAR-γA or D in their 5′ regions. hRAR-γC contains a 144 bp insertion (underlined in Fig. 6) between the residues corresponding to positions 272 and 273 of hRAR-γA. This insertion contains a termination codon (boxed in Fig. 6) in frame with the initiation codon of the common 454 amino-acid long ORF.

The last two cDNA clones, hRAR-γB and E, diverge from the other clones at a point which corresponds exactly to the boundary between the two exons which separately encode the A and B regions in both hRAR-α and hRAR-β [indicated by an arrowhead in Fig. 6; See , Brand, N., Petkovich, M., Krust, A., Chambon, P., de The, H., Marchio, A., Tiollais, P. & Dejean, A. Nature 332 , 850-853 (1988)]. However, these two clones are different from one another; the sequence of hRAR-γE diverges from those of all of the other forms of hRAR-γ cDNAs upstream from this point, whereas the corresponding sequence of hRAR-γB is colinear with the first 209 residues of hRAR-γA cDNA and with the first 95 residues of hRAR-γC (see Fig. 6). For all hRAR-γ cDNAs, except for hRAR-γC (see above), the common ORF remains open to their 5′ end which suggests the possible existence of several RAR-γ proteins, differing in their N-terminal region, in addition to the protein containing the common 454 amino acid-long sequence defined above.

## hRAR-γ acts as a retinoic acid-inducible transcription factor

To test the ability of hRAR-γ to activate transcription, hRAR-γD cDNA was subcloned into the eukaryotic expression vector pSG5 [Green, S., Issemann, I. & Scheer, E. Nucleic Acids Res. 16, 369 (1988)] as described in Example 6, to give hRAR-γO which was co-transfected into HeLa cells along with a reporter plasmid, $(TRE_3)_3$-tk-CAT, which contains a synthetic RA responsive element as described in Example 6. The transfected cells were exposed (24 hour post transfection) to increasing concentrations of RA, and RA-induced activation of transcription was evaluated 48 hours after transfection by determining the CAT activity expressed from the reporter gene. Fig 7 shows the CAT activity resulting from activation of the reporter gene $(TRE_3)_3$-tk-CAT by hRAR-γ0 in the presence of RA. Maximal transactivation was achieved at $10^{-8}$ to $10^{-7}$M RA, within a RA concentration range similar to that required to achieve maximal stimulation with expression vectors derived from hRAR-α and hRAR-β [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)], indicating that the cloned hRAR-γD cDNA encodes a functional retinoic acid receptor. As previously reported for hRAR-α and hRAR-β [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)], retinol was a much less potent activator than either all-trans or 13-cis retinoic acid (data not shown).

## hRAR-γ RNA is the predominant retinoic acid receptor RNA species in skin

The distribution of mRAR-α, β and γ mRNA in a variety of adult mouse tissues was compared above and it was shown that mRAR-α and mRAR-β were expressed in many of the tissues examined, whereas the expression of mRAR-γ RNA was almost exclusively restricted to skin (with very low levels of expression in lung and spleen). Northern blot analyses of total RNA extracted from adult human skin and poly(A)+ RNA prepared from fetal skin (Fig. 8, lanes 5 and 6, respectively), using [$^{32}$P]-labelled hRAR-α, β and γ cDNA probes under stringent conditions, showed that hRAR-γ RNA was also the predominant RAR RNA species in both adult and fetal human skin [the densitometric scanning of the reference actin (ACT) RNA signal indicated that there was 7-fold more actin mRNA in lane 6 than in lane 5]. hRAR-γ RNA may also be present in human lung (lane 7), albeit at a much lower level. The description of the Northern Blot Analysis is described in Example 7.

The expression of all three RAR RNAs in several human cell lines, including the breast cancer cell line T47D from which the hRAR-α and hRAR-γ cDNAs have been cloned (lane 8) was also studied. A comparatively high level of hRAR-γ RNA was seen in a human teratocarcinoma cell line (lane 2), which is reminiscent of a similar predominace of mRAR-γ RNA in the mouse F9 teratocarcinoma cell line. In contrast, expression of neither hRAR-γ nor hRAR-β was detected in the HepG2 human hepatoma cell line (lane 3), and the relative level of hRAR-γ was low in both the adenovirus transformed 293 cell line (lane 4) and a neuroblastoma cell line (lane 1). Note also that hRAR-α appears to be ubiquitously expressed, and that the ratio of the two subspecies of hRAR-α and hRAR-β RNA was variable amongst the different cell lines examined. The significance of these differences in size and abundance of hRAR-α and β transcripts remains to be established.

## The cognate members of human and mouse RARs are highly conserved

The structure of nuclear receptors can be divided into 6 discrete regions designated A-F, originally defined by amino acid sequence comparisons between various members of the family and by analysing the conservation across species of the primary structure of a given member of the nuclear receptor family ( See , Green, S. & Chambon, P. Trends Genet. 4 , 309-314 (1988) and refs therein). The three human RARs can be similarly divided ( See , Brand, N., Petkovich, M., Krust, A., Chambon, P., de The, H., Marchio, A., Tiollais, P. & Dejean, A. Nature 332 , 850-853 (1988) and Table 1) and aligned with their murine cognates (Fig. 1a and b). In all three cases the comparison of the human and mouse sequences of a given RAR indicates a high degree of conservation between each of the corresponding domains (Table 1, columns II, III and IV). Greater than 90% amino acid identity is apparent between each of the corresponding regions (A to F) of the human and murine homologs, except for region F of RAR-γ (column II, row F). The lower degree of conservation (58%) between regions F of mouse and man RARs is due to a frame-shift mutation in the hRARγ sequence corresponding to the end of region F (see above). A similar comparison, but between the three human RARs (columns V, VI and VII), reveals a high degree of homology between hRAR-α, β and γ only for the regions corresponding to the DNA binding (region C) and the retinoic acid binding (region E) domains. Most noteworthy is the almost complete divergence of regions A (compare in row A, columns V to VII with columns II to IV) and F (compare in row F, columns V to VII with columns II to IV). The overall conservation of region D is also lower between the three human RARs than between two corresponding members of the human and mouse subfamilies (compare columns V to VII with columns II to IV). In fact, as observed also in the case of the three mouse RARs (Zelent, A., Krust, A., Petkovich, M., Kastner, Ph. and Chambon, P., Nature, In Press), the homology is even lower for a 24 amino acid-long central section of region D (approximately 40% sequence similarity between all three human RARs).

It should be apparent that the foregoing techniques as well as other techniques known in the field of medicinal chemistry can be employed to assay for agonists and antagonists which bind specifically to all of the RAR's equally or to each one differentially. The substance can be an endogenous physiological substance or it can be a natural or synthetic drug. The RAR cDNA of interest can be expressed in microorganisms such as *E. coli* or yeast, as well as mammalian cells such as Hela. They can also be used to make a transgenic animal such as a mouse or rat. Any appropriate promoter can be used to construct the vector and said construction is not limited to only the examples set forth herein.

This invention also makes it possible to assay for an antagonist that inhibits the effect of an agonist, but has no biological activity of its own in the RAR effector system. Thus, for example, the invention can be employed to assay for a natural or synthetic substance that competes for the same receptor site on the RAR protein or the DNA recognition site that the receptor occupies, or the invention can be employed to assay for a substance that can act on an allosteric site, which may result in allosteric inhibition or activation.

It will be understood that this invention is not limited to assaying for substances that interact only in a particular way, but rather the invention is applicable to assaying for natural or synthetic substances, which can act on one or more of the receptor or recognition sites, including agonist binding sites, competitive antagonist binding sites (accessory sites), and non-competitive antagonist or regulatory binding sites (allosteric sites).

A convenient procedure for carrying out the method of the invention involves assaying a system for stimulation of RAR by a retinoid. For instance, as a retinoid binds to the receptor, the receptor-ligand complex will bind to the responsive promoter sequences and will activate transcription. For example, levels of transcription of β-galactosidase or CAT genes can be determined and these genes can be used as reporter genes to test the activation potential of the RAR gene constructed therewith. The method of this invention makes it possible to screen various receptor binding retinoids. In addition, this invention makes it possible to carry out blood tests or tissue biopsies for RAR activity in patients. Any of the mouse RARs, α, β or γ, or human RAR γ molecules of the present invention can be used in the above.

The cDNAs encoding these receptors can be used as probes to determine the state of expression of each of these receptors in normal or diseased tissues or cultures.

Cloning of alternatively spliced mRAR-γ cDNAs

Using the cloned mRAR-γ cDNA described above as a probe, 6 clones (F9.1 to 6) were isolated from two λZAPII cDNA libraries of untreated or RA-treated (48 hours) F9 EC cells. These cDNA libraries were constructed from polyA$^+$ RNA in a λZAPII vector according to the procedure recommended by the manufacturer (Stratagene). Clones hybridizing under strigent conditions with mRAR-γ cDNA were sequenced using the universal primer, the reverse primer and the mRAR-γ C region antisense primer.

Positive clones fell into three categories (mRAR-γ1, γ2 and γ3) according to the sequence upstream from the splice junction separating the exons encoding the A and B regions (A/B junction). The mRAR-γ1 sequence (clones F9.1 and 2; Fig. 9a) was colinear with that of the previous mRAR-γ cDNA described above, but had 150 additional 5′ nucleotides and a CAG triplet inserted at position 183 (boxed in Fig. 9a). mRAR-γ1 shared extensive homology with the human RAR-γA described above, including the 5′-UTR (Fig. 9a). Interestingly, a similar CAG insertion exists at the corresponding position of the hRAR-γF cDNA clone isolated from a T47D cDNA library and otherwise colinear with hRAR-γA. Southern blot analysis and DNA sequencing of a λ genomic DNA clone encoding the 5′ region of the mRAR-γ gene (λG1mRAR-γ, Fig. 9i) indicated that the 5′-UTR and A region (A1, Fig. 9a) of mRAR-γ1 are encoded in a minimum of three exons (E1,E2 and E3; see Fig. 9a and 9g in which the location of exon junctions 1, 2 and 3 are also indicated; note that E1 may correspond to more than one exon).

The mRAR-γ2 cDNA (clones F9.3 to 5) possessed sequences upstream of the A/B junction which were different from those of mRAR-γ1. These and additional 5′ sequences obtained by anchor PCR (see Example 8) revealed the presence of an ATG in frame with the common mRAR-γ ORF. Furthermore, the F9.5 clone possessed this common ORF in its entirety, thus indicating the mRAR-γ2 cDNA encodes a putative mRAR-γ2 protein which differs from mRAR-γ1 only in its A region (A2 in Fig. 9b) and 5′-UTR. These two regions are encoded in exon E5 which is also present in λG1mRAR-γ (Fig. 9i; see Fig. 9b and 9g). Interestingly, the mRAR-γ2 sequence immediately upstream of the A/B junction was similar to that of the human hRAR-γE cDNA clone described above. Additional sequencing of the 5′-UTR and A region of hRAR-γ2 (using clones obtained by anchor PCR on RNA from human fetal skin) revealed that they were both highly homologous to their mouse counterparts (Fig. 9b).

The mRAR-γ3 sequence upstream of the A/B junction (Fig. 9c) was established using the F9.6 clone and two clones (11.5d/1 and 11.5d/2) isolated from an 11.5 day embryo λgt10 cDNA library as described above. The 11.5d/1 clone possessed the ORF encoding the mRAR-γ regions B to F. Since the mRAR-γ3 sequence contains an upstream ATG (boxed in Fig. 9c) in frame with the common mRAR-γ ORF, the 11.5d/1 clone may correspond to an additional mRAR-γ protein with an alternative A region. However, sequencing of λG1mRAR-γ upstream from exon E8 (Fig. 9i) indicated that the 5′ mRAR-γ3 sequence corresponds to the intronic sequence immediately upstream of exon 8 (note also a mouse B1 repetitive sequence located between positions 222 and 308). Furthermore, the human genomic sequences upstream of exon 8 did not reveal any significant conservation (Fig. 9c), which suggests that the mRAR-γ3 cDNA clones correspond to partially spliced primary transcripts.

Additional mRAR-γ cDNA clones possessing alternative upstream sequences were obtained using anchor PCR (see Example 8) with polyA$^+$ RNA isolated from mouse new-born skin, 8.5, 9.5, 11.5 and 14.5 days embryos, and P19 and F9 EC cells. Sequences corresponding to mRAR-γ1, γ2 and γ3 were again found, with mRAR-γ1 and γ2 accounting for about 90% of the clones irrespective of the origin of the RNA. Four additional types of clones were obtained (mRAR-γ4 to 7) with sequences diverging from those of mRAR-γ 1 to 3, and from each other, exactly at the A/B splice junction, upstream of exon 8 (Fig. 9).

The original mRAR-γ4 clones were isolated from 8.5 day (one clone) and 9.5 day (one clone) embryo RNA. Further 5′ sequences were obtained using anchor PCR with mRAR-γ4-specific 3′ primers (Fig. 9d). A variability in the length of the polydC stretch (9-11 residues; underlined in Fig. 9d) was observed. The clone containing 11 C residues has an ATG in frame with the mRAR-γ common ORF (boxes in Fig. 9d), thus conceptually encoding an additional mRAR-γ protein with an alternative A region. It should be noted, however, that only 7 C residues were found in exon 6, which contains the corresponding sequence in the genomic DNA clone λG1mRAR-γ. Whether this variability reflects a true mouse polymorphism or corresponds to cloning artifacts is unknown. In any case, mRAR-γ4 in which exon 6 is spliced to exon 8 cannot be a partially spliced pre-mRNA (see Fig. 9i and g). PCR amplification using polyA$^+$ RNA from 9.5 day embryos, a 5′ primer specific for mRAR-γ4 and a 3′ primer specific to the common mRAR-γ 3′-UTR, enabled us to demonstrate the existence of mRAR-γ4 transcripts possessing the entire B to F mRAR-γ ORF.

mRAR-γ5 (Fig. 9e) and mRAR-γ6 (Fig. 9f) clones were obtained from RNA of RA-treated F9 cells, and from RNA of skin, F9 and P19 cells, respectively. Both isoforms possess an in-frame stop codon upstream of the common mRAR-γ ORF. PCR amplication using RNAs from F9 cells, 5′ primers specific for either mRAR-γ5 or mRAR-γ6, and a 3′ primer corresponding to the common mRAR-γ 3′-UTR, established the presence of mRAR-γ5 and mRAR-γ6 transcripts colinear with the mRAR-γ B to F ORF. Thus the mRNA's corresponding to mRAR-γ5 and γ6 probably encode identical proteins lacking an A region, with an N-terminal methionine corresponding to the first ATG of exon 8 (Fig. 9g). However, they differ in their 5′-UTRs which are encoded in two separate exons (4 for mRAR-γ5, and 7 for mRAR-γ6) of λG1mRAR-γ (see Fig. 9i and 9g).

mRAR-γ7 cDNA (Fig. 9g) was isolated using 9.5 day embryo RNA. Interestingly, its sequence upstream of the A/B junction was identical up to its very 5′-end to that of the first 255 bases of mRAR-γ1 encoded in exons 1 and 2 (Fig. 9g). Thus mRAR-γ7 results from an alternative splicing which joins exon 2 to exon 8, whereas in mRAR-γ1 exon 2 is joined to exon 3. There is no in frame ATG, nor stop codon upstream of exon 8 in the mRAR-γ7 cDNA sequence, which indicates that the corresponding mRNA may encode a mRAR-γ protein lacking an A region. Again PCR amplification carried out as above established the existence of mRAR-γ7 transcripts containing the mRAR-γ B to F ORF.

These results indicate that the above described mouse RAR-γ cDNA isoforms all differ in their sequences upstream from the point corresponding exactly to the 5′ end of exon 8 which encodes the B region (Fig. 9i; arrowheads 3 and 4-7 in Fig. 9a-f). In contrast, the mRAR-γ B-F regions appear to be common to all of these isoforms. These differing 5′-UTR and A regions are generated by alternative splicing of at least 7 exons (E1 to E7 in Fig. 9) which span at least 14.2 kb (the distance between exons E2 and E8; exon E1 has not yet been mapped). mRAR-γ1 (mRAR-γ described above) and mRAR-γ2 mRNAs encode two mRAR-γ isoforms which differ in the sequence of their A regions (see Fig. 9) and 5′-UTR's. Moreover, both mRAR-γ1 mRNA (see above discussion) and mRAR-γ2 mRNA encode receptors which act as transcriptional RA-inducible enhancer factors. mRAR-γ5, γ6 and γ7 (and possibly mRAR-γ4) mRNAs all appear to encode the same putative mRAR-γ protein which does not possess an A region and is truncated for the first 11 amino acids of region B (see Fig. 9). However, all four mRAR-γ4 to γ7 mRNAs differ in their 5′-UTR's which are encoded in different exons.

From the above discussion, it is readily apparent that the nucleotide and deduced amino acid sequences of the various mRAR-γ isoforms can be derived from Figures 1 and 9 of the present specification. The nucleotide sequence encoding mRAR-γ1 comprises the nucleotides from Figure 9a (5′) and nucleotides 538-1912 from Figure 1c (3′), while the amino acid sequence of mRAR-γ1 comprises the amino acid sequence shown in Figure 1c. The nucleotide sequence encoding mRAR-γ2 comprises the nucleotides from Figure 9b (5′) and nucleotides 538-1912 from Figure 1c (3′), while the amino acid sequence of mRAR-γ2 comprises the amino acids from Figure 9b (N-terminal), and amino acids 75-458 from Figure 1c (C-terminal). The nucleotide sequence encoding mRAR-γ3 comprises the nucleotides from Figure 9c (5′) and nucleotides 539-1912 from Figure 1c (3′). The nucleotide sequence encoding mRAR-γ4 comprises the nucleotides from Figure 9d (5′) and nucleotides 542-1912 from Figure 1c (3′), while the amino acid sequence of mRAR-γ4 comprises amino acids 73-458 from Figure 1c. The nucleotide sequence encoding mRAR-γ5 comprises the nucleotides from Figure 9e (5′) and nucleotides 551-1912 from Figure 1c (3′), while the amino acid sequence of mRAR-γ4 comprises amino acids 73-458 from Figure 1c. The nucleotide sequence encoding mRAR-γ6 comprises the nucleotides from Figure 9f (5′) and nucleotides 546-1912 from Figure 1c (3′), while the amino acid sequence of mRAR-γ4 comprises amino acids 73-458 from Figure 1c. Finally, the nucleotide sequence of mRAR-γ7 comprises nucleotides 1-255 from Figure 9a (5′) and nucleotides 500-1912 from Figure 1c (3′), while the amino acid sequence of mRAR-γ7 comprises amino acids 73-458 from Figure 1c. Of course, due to the degeneracy of the genetic code, nucleotide sequences other than those described above can encode the corresponding amino acid sequences.

<u>Human homologs to mRAR-γ cDNA isoforms</u>

As noted above, there is a high degree of conservation between mRAR-γ1 and γ2 and their respective human counterparts (hRAR-γA and γE). This homology encompasses both the protein-coding region A and the 5′-UTR (>90% homology in these regions), indicating that the counterparts of mRAR-γ exons E1, E2, E3 and E5 are well conserved in the human genome and that both regions must be functionally important.

The conservation of mRAR-γ exons E6 and E7 present in mRAR-γ4, and γ6, respectively, was investigated by comparative Southern blot analysis of human and mouse genomic DNA, using probes specific for these two mouse exons (101-267 and 1-373 for mRAR-γ4 and γ6, respectively, see Fig. 9d and f). In both cases, a specific cross-hybridization was observed with human genomic DNA restriction digests, indicating the possible existence of human counterparts to mRAR-γ4 and γ6 isoforms.

In addition to the already mentioned hRAR-γA, γE and γF cDNAs, three T47D cell hRAR-γ cDNAs with sequences diverging upstream of the A/B junction (hRAR-γB, γC and γD; see Fig. 9h) have been described. Sequence comparisons indicate that hRAR-γB corresponds to an alternative splicing between the human counterparts of mRAR-γ exons E1 and E8. hRAR-γC is identical to hRAR-γA (mRAR-γ1 counterpart), but contains an additional sequence inserted between the exonic sequences E2 and E3 (Fig. 9g and h), which suggests that there could be an additional mouse exon (designated here EC) located between E2 and E3. The existence of human isoforms hRAR-γB and γC suggests that at least two additional mRAR-γ isoforms remain to be discovered.

<u>Specific pattern of expression of mRAR-γ isoforms</u>

Probes specific to mRAR-γ1, γ2, γ3, γ4, γ5 and γ6 isoforms were used to investigate their expression in different adult mouse tissues, embryo stages and RA-responsive ES and EC cells (Fig. 10a). mRAR-γ1 RNA was readily detected as an approximately 3.3 kb transcript on Northern blots from adult lung, EC and ES cells, and total embryos at various stages, but the highest expression was observed in adult and new-born skin. The mRAR-γ2 specific probe revealed a shorter 3.1 kb transcript, which was expressed at a much lower level in skin and roughly at similar levels as mRAR-γ1 RNA in lung, EC and ES cells, and embryos. A single 6.6 kb transcript was detected in EC cells (arrowhead) using the mRAR-γ6 probe. Whether this transcript corresponds to a mature mRNA-γ6 mRNA or to a partially processed pre-mRNA remains to be seen. The mRAR-γ4 and γ5 probes did not reveal any hybridizing RNA species in the samples examined, even on overexposed blots. In contrast, the mRAR-γ3 probe detected an approximately 1.6 kb transcript in RNA from adult liver, brain of 16.5 day embryo, and 14.5 day total embryo. This 1.6 kb RNA, which was not detected with the total mRAR-γ cDNA probe described above, is transcribed from the antisense strand. No additional transcript hybridizing with the mRAR-γ3 probe was detected, except a 6.6 kb RNA in F9 EC cells, indicating that mRAR-γ3 could correspond to a partially processed RNA (see above). The approximately 14.5 kb transcripts which are detected with all probes in skin (black arrowhead), may also represent partially processed transcripts.

Clearly, mRAR-γ1 and γ2 are the predominant mRAR-γ forms in all mouse cells, tissues and embryos which have been analyzed. To examine more quantitatively the relative abundance of mRAR-γ1 and γ2 transcripts in the various RNA preparations, RNase protection assays were performed with RNA probes spanning the common B regicn and either the A1 (Fig. 10b and d) or the A2 region (Fig. 10c and e). The relative amount of the other RAR-γ RNAs [mRAR-γ(Σ-1) and mRAR-γ(Σ-2) in Fig. 10b to e] was also quantified in each case. The amount of mRAR-γ1 and mRAR-γ2 relative to total mRAR-γ (mRAR-γΣ) is indicated at the bottom of panels d and e. mRAR-γ1 was the predominant RAR-γ isoform in skin (≧90%), whereas mRAR-γ2 was very low (<5%) in this tissue. In contrast, mRAR-γ2 is the predominant isoform in F9 and ES cells. Both isoforms were represented in the embryo, but there was a relative decrease in mRAR-γ2 transcripts during the course of embryogenesis, with a concomitant increase of mRAR-γ1 transcripts (compare lanes 8-12 in Fig 10d and e). In other words, mRAR-γ2 expression is predominant in early embryos (8.5 and 9.5 day embryos), while mRAR-γ1 mRAR becomes predominant at later stages of development (11.5 to 14.5 day embryos).

## Brief Description of Figures

Figure 1 shows amino acid sequences of mRAR-α (a) and β (b), and nucleic acid and deduced amino acid sequences of mRAR-γ (c).

Figure 2 shows amino acid alignment of the mRAR-α, β and γ (as indicated).

Figure 3 shows RA-dependent transcriptional transactivation by mRARs transiently expressed in HeLa cells.

Figure 4 shows Northern blot analysis of mRAR-α, β and γ poly(A) RNA from various mouse tissues (as indicated in a, lanes 1-14), and F9 teratocarcinoma cells (b, lanes 15-17) before (lane 15), and after 24 hours (lane 16) and 48 hours (lane 17) treatment with RA ($3.3x10^{-7}$M).

Figure 5 shows hRAR-γ cDNA sequence and comparison of its deduced amino acid sequence with that of mRAR-γ.

Figure 6 shows comparison of the 5′ regions of five hRAR-γ cDNAs.

Figure 7 shows RA-dependent transcriptional activation by hRAR-γ0.

Figure 8 shows northern blot analysis of hRAR-α,β and γ RNAs from various tissues and cells.

Figure 9 shows the amino acid and nucleotide sequence analysis of mouse RAR-γ isoforms.

Figure 10 shows analysis of the distribution of mRAR-γ transcripts in various mouse tissues.

## Example 1

## Method of determining amino acid and nucleic acid sequence

Approximately 2 million recombinant phages from an 11.5 day randomly primed mouse embryo λgt10 cDNA library (a gift of B. Galliot and D. Duboule) were screened with purified hRAR-α and β cDNA probes derived from clones hRAR-αO and hRAR-β) [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)] and [$^{32}$P]-labelled by random priming [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)] to specific activity of.approximately $10^9$ cpm/μg of DNA. Plaque lifts onto nitrocellulose filters and treatment of the filters before hybridization were as described [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)]. Hybridization was carried out at 37°C for approximately 24 hours in 5xSSPE (0.75 M NaCl 50 mM Na $H_2PO_4$ 5mM EDTA, pH 7.4), 40% formamide, 0.2 mg/ml sheared and denatured salmon sperm DNA, and 1X Denhardt's reagent [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)]. The most stringent wash was done in 2xSSPE./0.1 % SDS/0.06 % sodium pyrophosphate (NaPPi) at 50°C for 20 minutes. Filters were exposed for 36 hours at -80°C (one intensifying screen, Kodak XRO-5 film). Positive clones were rescreened under the same conditions and phage DNA was prepared from the clones which remained positive. These clones were divided into three groups based on Southern blot hybridization data with hRAR-α and β cDNA probes and restriction enzyme digest patterns. Selected cDNAs from each group were subcloned into either pTZ19R, or pEMBL18 and 19 vectors, single stranded DNA was prepared [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)], and sequenced using the dideoxy chain termination procedure [Sanger, F., Nicklen, S. & Coulson, A. R. Proc. Natl. Acad. Sci. USA 74 , 5463-5468 (1977)]. Two clones which contained the entire open reading frame of mRAR-γ and mRAR-β were sequenced either on both strands (mRNA-γ, sequence shown in panel c), or one strand (mRNA-β, panel b, any ambiguity was resolved by seqencing on the other strand). The mRAR-α sequence was derived from two clones which were sequenced on both strands and overlapped between amino acids 64 and 283 of the sequence displayed in fig. 1 A mRAR-α cDNA clone containing the entire open reading frame was subsequently constructed in pSG5 [Green, S., Issemann, I. & Scheer E. Nucleic Acids Res. 16, 369 (1988)] by using the unique Bsu36I site located in the DNA sequence encoding amino acids 182-184.

## Example 2

### Transfection of HeLa Cells

HeLa cells (~ $10^6$ per dish) were cotransfected with 0.5 μg of a given mRAR expression vector, 2 μg of reporter plasmid and 2 μg of pCH110 (Pharmacia, a β-galactosidase expression vector used as an internal control to normalize for variations in transfection efficiency). The total amount of transfected DNA was adjusted to 20μg by addition of carrier DNA (BSM13+). Cell culture media, treatment of cells, preparation of cytosolic extracts, and CAT assays were carried out as previously described [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)]. The reporter plasmid $(TRE_3)_3$-tk-CAT (de Verneuil, H., Metzger, D. & Chambon, P., in preparation) contains a trimer of a synthetic RARE (5′-AGCTTAGGTCAGGGACGTGACCTT-3′) inserted in pBLCAT8 [Klein-Hitpass, L., Ryffel, G. U., Heitlinger, E. & Cato, A. C. B. Nucleic Acids Res. 16 , 647-663 (1988)] upstream of the tk promoter. mRAR-αO was constructed by inserting the reconstructed mRAR-α cDNA (see Fig. 1) into the EcoRI site of pSG5 [Green, S., Issemann, I. & Scheer, E. Nucleic Acids Res. 16 , 369 (1988)], mRAR-βO was obtained by inserting the Eag1/BaMHI fragment of mRAR-β cDNA into the BaMHI site of pSG5 with the help of a BaMHI/Eag1 adaptor, and mRAR-γO was constructed by ligating the EcoRI-flanked mRAR-γ cDNA (Fig. 1c) into the EcoRI site of pSG5.

## Example 3

### RNA Isolation and Northern Blot Analysis

RNA was extracted from various mouse organs and cells using the GnSCN-CsC1 procedure [Chirgwin, J. M., Przybyla, A. E., MacDonald, R. J. & Rutter, W. J. Biochemistry 18 , 5294-5299 (1974)]. Poly(A)+ RNA was prepared by oligo (dT)-cellulose chromatography [Aviv, H., Leder, P. Proc. Natl. Acad. Sci. USA 69 , 1408-1412 (1972)], electrophoresed on 1 % agarose-1.1M formaldehyde gels [Lehrach, H., Diamond, D., Wozney, J. M. and Boedtker, H. Biochemistry 16 , 4743-4751 (1977)], and transferred to nitrocellulose filters using standard protocols [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)]. Hybridization conditions were essentially the same as in example 1, except that 50% formamide was used and hybridization was carried out at 42-45°C (37°C-40°C for oligonucleotide probes) for approximately 18 hours. Filters were dehybridized by 5 min. treatment in 0.05 x SSPE at 90°C. The specific activity of all random-primed cDNA probes and labelled oligonucleotides was consistently approximately $10^9$ and $10^8$ cpm/μg DNA, respectively, The most stringent wash (15 minutes) was at 65°C in 0.1xSSPE/1.0%SDS/0.03% NAPPi for filters hybridized with randomly primed cDNA probes and at 55°C in 1xSSPE/1.0%SDS/0.03%NaPPi with [$^{32}$P]-end labelled oligonucleotide probes.

Example 4

Cloning and sequencing of hRAR-γ cDNA

Approximately $10^6$ phage from T47D cell breast cancer λgt11 (μg) and λgt10 (a gift of J. M. Garnier) libraries were screened with a [$^{32}$P]-labelled nick translated cDNA probe [Petkovich, M., Brand, N. J., Krust, A. & Chambon, P. Nature 330 , 444-450 (1987)] encompassing the entire mRAR-γ ORF as described above. 8 clones were isolated which gave strong hybridization signals after two rounds of screening. cDNA inserts were subcloned into the EcoRI site of pEMBLI9+ [Petkovich, M., Brand, N. J., Krust, A. & Chambon, P. Nature 330 , 444-450 (1987)] and sequenced using the dideoxy technique on both strands [Sanger, F., Nicklen, S. & Coulson, A. R. Proc. Natl. Acad. Sci. USA 74 , 5463-5468 (1977)] with synthetic oligonucleotide primers.

Example 5

RNA isolation and Northern analysis

Poly(A)+ RNA was isolated and electrophoresed through a 1% agarose formaldehyde gel as described [Chirgwin, J. M., Przybyla, A. E., MacDonald, R. J. & Rutter, W. J. Biochemistry 18 , 5294-5299 (1974)]. The gels were blotted onto nitrocellulose filters (Schleicher and Schull BA85) and hybridization and washing of the filters under stringent conditions with randomly primed [$^{32}$P]-labelled probes derived from hRAR-α, β and γ cDNA were performed as described [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)]. Autoradiography was performed with Kodak XAR-5 films at -80 °C and intensifying screens.

Example 6

Transcriptional activation by hRAR-γ0

The entire 1.5 kb insert from the hRAR-γD clone was inserted into the EcoRI site of the eukaryotic expression vector pSG5 [Green, S., Issemann, I. & Scheer, E. Nucleic Acids Res. 16 , 369 (1988)], to yield hRAR-γ0. 500 ng of hRAR-γ0 was transfected into HeLa cells along with 2 μg of the $(TRE_3)_3$-tk-CAT reporter plasmid, 2 μg of a β-galactosidase expressing plasmid pCH110 (to normalize for variations in transfection efficiency) and 15 μg of carrier DNA (BSM13+) as described [Petkovich, M., Brand, N. J., Krust, A. & Chambon, P. Nature 330 , 444-450 (1987)]. 24 hours following transfection, the cells were exposed for an additional 24 hour to RA for retinol with concentrations ranging from $10^{-11}$M to $10^{-6}$. Extracts of HeLa cells were prepared and assayed for CAT activity as described [Petkovich, M., Brand, N. J., Krust, A. & Chambon, P. Nature 330 , 444-450 (1987)].

Example 7

Northern blot analysis of hRAR-α, β and RNAs from various tissues and cells

RNA preparation blotting, electrophoresis and hybridization with [$^{32}$P]-labelled cDNA probes were as described above. Lanes 1-4: 7 μg each of poly-(A)+ RNA prepared from human neuroblastoma cells IMR32 [Tumilowicz, J. J., Nichols, W. W., Cholon, J. J. and Greene, A. E. (1970) Cancer Research 30 , 2110-2118], human teratocarcinoma cells T2CL13 [Weima, S. M., van Rooijen, M. A., Mummery, C. L., Feijen, A., Kruijer, W., de Laat, S. W. and van Zoelen, E. J. J. (1988) Differentiation 38 , 293-210], human hepatoma cells HepG2 [Knowles, B., Howe, C. C. and Aden, D. P. (1980) Science 209 , 497-499] and human adenovirus transformed cells 293 [Graham, F., Smiley, J., Russel, W. C. and Narin, B. (1977) J. Gen. Virol. 36 , 59-72] as indicated; lanes 5: 20 μg of total RNA extracted from adult human skin; lanes 6 and 7; 4 μg each of poly(A+) RNA from human fetal skin and adult lung as indicated; lane 8: 4 μg of poly(A)+ RNA from T47D breast cancer cells. Autoradiography was for 14 hours, 7 days and 48 hours with hRAR-α, β and γ cDNA probes, respectively. Blots were rehybridized with an actin cDNA probe to check the integrity of the RNA preparation. Sizes shown on the right in kb for the RAR RNA species were calculated from their migration relative to the 28S and 18S rRNAs whose sizes are 4712 and 1869 nucleotides, respectively [Hassouna, N., Michot, B. & Bachellerie, J. P. Nucleic Acids Res. 12 , 3563-3583 (1984); Raynal, F., Michot, B. &

Bachellerie, J. P. FEBS Letters 167 , 263-268 (1984); McCallum, F. S. and Maden, E. H. (1985) Biochem. J. 232 , 725-733].

## Example 8

### Anchor PCR and other methodology used in mRAR-γ Isoform studies

Anchor PCR amplification starting from RNA was performed as described [Loh, E.Y., Elliott, J.F., Cwirla, S., Lanier, L.L. & Davis, M.M. (1989) Science 243 , 217-220] using single-stranded cDNA synthesized from 1 µg of polyA$^+$ RNA and primed with the mRAR-γ C region antisense oligonucleotide 5′-CCATAGTGGTAGCC. Nucleotides were removed by Sephadex G50 filtration and the cDNA was G-tailed as described [Deng, G. and Wu, R. (1984.) Methods in Enzymol 100 , 96]. 2 rounds of 20-30 PCR cycles were then carried out using successively two appropriate mRAR-γ 3′ primers and the same 5′ anchor primers as in Loh, E.Y., Elliott, J.F., Cwirla, S., Lanier, L.L. & Davis, M.M. (1989) Science 243 , 217-220. Prior to the second round of amplification, molecules larger than 200 bp were excised from a 2% agarose gel, purified by Geneclean, and 1/10 of the material was further amplified using the second 3′ primer which carried either BamHI, HindIII and/or XbaI sites. Following directional cloning between one of these sites and Not1 of the Bluescript SK+ vector, the DNA from positive colonies was sequenced. Anchor PCR on genomic DNA was performed as above, except that the first strand synthesis was performed on 1 µg of denatured genomic DNA (1 unit of Taq polymerase, 5 min at 55 °C).

All other techniques used in the identification and characterization of the mRAR-γ isoforms are described herein or in Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987).

## Table 1

### Amino acid sequence similarities between human and mouse retinoic acid receptors

The amino acid sequences of regions A to F (column I) previously defined for hRAR-α and β - [Brand, N., Petkovich, M., Krust, A., Chambon, P., de The, H., Marchio, A., Tiollais, P. & Dejean, A. Nature 332 , 850-853 (1988)], mRAR-α, β and γ and in Fig. 5 for hRAR-γ were compared. For each set of comparisons the percentage of amino acid identity (after optimal alignment) is shown (column II-VII). Column VIII corresponds to similar comparisons between regions A-F of the human [Krust, A., Green, S., Argos, P., Kumar, V., Walter, P., Bornert, J. M. and Chambon, P. (1986) EMBO J. 5 , 891-897] and mouse [White, R., Lees, J. A., Needham, M., Ham, J. & Parker, M. Molec. Endocrin. 1 , 735-744 (1987)] oestrogen receptors. hα, β and γ, and mα, m β and γ correspond to hRAR-α, β and γ, and mRAR-α, β and γ, respectively.

## DISCUSSION

cDNA clones were isolated from a human T47D cell library which contain a common (open reading frame ORF) encoding a protein highly homologous to the above characterized mouse RAR-γ. That this ORF encodes a functional retinoic acid receptor was established by its transient expression in HeLa cells, where it could activate the transcription of a RA-responsive reporter gene within a range of RA concentration similar to that reported for transcriptional transactivation by the different members of the human [Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988)] and mouse RAR sub-family. This brings to three the number of human retinoic acid receptors characterized to date.

The extensive amino acid sequence homology between hRAR-γ and mRAR-γ suggests strongly that they are functionally equivalent. In this respect, we note that hRAR-γ gene is located on chromosome 12, whereas the mRAR-γ gene is located on the mouse chromosome counterpart, chromosome 15. The comparison of the amino acid sequences of human and mouse RARs also suggests strongly that hRAR-α and mRAR-α, and hRAR-β and mRAR-β, are functionally equivalent. It is particularly striking that the A, B, D and F regions are highly conserved between man and mouse for a given member of the RAR subfamily, whereas they are not, or to a much lesser extent, when comparing the different RARs of either man or mouse (Table 1). These regions are usually not as well conserved across species for a given steroid hormone receptor (see Table 1, column VIII, for a comparison between the human and mouse oestrogen receptors). The almost complete conservation across species in the case of each of the three members of the RAR subfamily indicates that these regions must be important for their functional specificity. Since the DNA-binding domains (regions C) of RAR-α, β and γ are highly conserved, all three receptors may interact with the same responsive elements. The A/B regions of the oestrogen and progesterone receptors appear to play a role in specific transcriptional transactivation of some target genes (Green, S. & Chambon, P. Trends Genet. 4 , 309-314 (1988) and Tora, L., Gronemeyer, H., Turcotte, B., Gaub, M. P. & Chambon, P. Nature 333 , 185-188 (1988) and refs therein). The different A/B regions of the three RARs may have a similar function. The lower conservation for the F region of human and mouse RAR-γ is due to a single base difference in the region encoding the last amino acids of this region. The same single base difference was found in two independent mRAR-γ cDNA clones, and in all three hRAR-γ cDNA clones which contain this region (see above). Moreover we can exclude the possibility that the T47D breast cancer cell hRAR-γ gene bears a mutation not present in normal cells, since the same sequence was found in cDNA derived from skin hRAR-γ mRNA and amplified using the polymerase chain reaction (data not shown). Several hRAR-γ cDNA clones that we have analyzed here differ in their 5′ region. In two cases (hRAR-γB and E) these differences may well reflect alternative splicing, since the point of divergence is located at a position which is known to correspond to the boundary between the exons separately encoding regions A and B of both hRAR-α (N. Brand and P. Chambon, unpublished results) and hRAR-β ( See , Petkovich, M., Brand, N. J., Krust, A. & Chambon, P. Nature 330 , 444-450 (1987); Brand, N., Petkovich, M., Krust, A., Chambon, P., de The, H., Marchio, A., Tiollais, P. & Dejean, A. Nature 332 , 850-853 (1988); Dejean, A., Bougueleret, L., Grzeschik, K-H. and Tiollais, P. (1986) Nature 322 , 70-72). We note that a similar situation may exist in the case of the thyroid hormone receptors for which two β forms diverging at the corresponding exon-intron boundary (Zahraoui, A. and Cuny, G. (1987) Eur. J. Biochem. 166 , 63-69) have been recently described (Hodin, R. A., Lazar, M. A. Wintman, B. I., Darling, D., Koenig, R J., Larsen, P. R., Moore, D. D. and Chin, W. W. (1989) Science 244 , 76-79). Whether alternative splicing is also responsible for the other differences seen in the hRAR cDNA 5′ regions is unknown. Since the hRAR-γ major ORF remains open up to the 5′ end in four out of five cDNA clones that we have sequenced here, it is possible that there are several hRAR-γ proteins which differ in their N-terminal sequence, and thus exhibit different target gene specificity (see above).

We have shown herein that mouse RAR-γ RNA is expressed at a much higher level in skin than in any other adult tissue analyzed. hRAR-γ RNA is also the predominant RAR RNA speoies in human adult and fetal skin (Fig. 8). This observation is particularly interesting in view of the effect of retinoids on skin, both in normal and pathological states. That hRAR-γ RNA is the predominant RAR species in a human teratocarcinoma (Fig. 8) suggests that hRAR-γ, along with hRAR-α and β, may also play a role during embryogenesis.

It has been thought that the range and diversity of effects of RA would preclude the possibility that a single molecular mechanism might account for all of them. Direct control of gene expression by multiple RA receptors may however account for a large proportion of the RA effects, provided that the various RARs would exhibit different spatial and temporal patterns of expression and would specifically activate different target genes.

## DETAILED DESCRIPTION OF FIGURES

Figure 9: Mouse RAR-γ isoforms. (a) Alignment of the 5′ sequences of mouse (m) and human (h, top lines) RAR-γ1 (ie., hRAR-γA) cDNA's. Gaps have been introduced for optimal alignment, bases differing in hRAR-γ are given and conserved bases are indicated by dashes. Amino acids which differ in the A1 region between mRAR-γ1 and hRAR-γ1 are indicated. Region A1 is boxed, an in-frame stop codon and the beginning of the B region are underlined. The CAG insertion (see text) is boxed. Filled triangles indicate exon boundaries 1-3 as deduced from sequences of λG1mRAR-γ (panel i). (b) Alignment of the 5′ sequences of mRAR-γ2 and hRAR-γ2 (ie., hRAR-γE). (c) 5′ sequences of mRAR-γ3 cDNA. An ATG codon in frame with the common mRAR-γ ORF is boxed, the preceding in frame stop codon is underlined. Human genomic sequence upstream of the B region of the hRAR-γ gene have been determined by anchor PCR and aligned with the mouse sequence (given on top; same representation as in 9a and 9b)

↑ indicates the 5' limit of the exon encoding region B. (d) 5′ sequences of mRAR-γ4 cDNA. The stretch of 11 C's is underlined. An ATG codon in frame with the common RAR-γ ORF is boxed. (e) and (f) 5′ sequences of mRAR-γ5 and mRAR-γ6 cDNAs, respectively. (g) mRAR-γ isoforms. The general organization of the mRAR-γ's is depicted at the top and mRAR-γ1 to γ7, as deduced from sequencing data (panels a to f), are schematized below. The ORF common to all isoforms is indicated by a line spanning regions B to F. Arrowheads indicate boundaries between exons (E1 to E8). (h) Schematic representation of hRAR-γ isoforms. The representation is the same as in panel g. Clones hRAR-γB and hRAR-γC are described above. Splicing boundaries (arrowheads) are inferred from knowledge of the structural organization of mRAR-γ's. The additional exon present in hRAR-γC is called EC (see text). (i) Schematic representation of the 5′ region of mRAR-γ gene. The genomic clone λG1mRAR-γ was obtained by screening an L cell genomic library with a 5′ mRAR-γ1 probe. BamHI (B), EcoRI (E) and KpnI (K) restriction sites, and the approximate location of the exons (panel g) are indicated. The location of the putative mouse EC exon is also indicated. Exon E1 (in parenthesis) has not yet been mapped. With the exception of exon E6 (see text) the exonic sequences were identical in mRAR-γ cDNA isoforms and λG1mRAR-γ.

Figure 10 : Distribution of mRAR-γ transcripts. (a) Northern Blot analysis. 4 μg of polyA+ RNA prepared from adult (A) and new-born (N) tissues, untreated (-) or RA-treated (+) EC cells, total embryos collected at various days (D) postcoitum and 16.5 day embryonic brain (BR), or 15 μg of total RNA prepared from ES cells untreated (-) or RA-treated (+), were loaded on a 1% formaldehyde gel, as indicated. Blots were hybridized with probes prepared by PCR (Scholwater, D.B. and Sommer, S.S. (1989) Anal. Biochem. 177 , 90-94) and specific for mRAR-γ1, mRAR-γ2, mRAR-γ3, and mRAR-γ6. An actin cDNA probe was used to check the integrity of RNA as described above. Positions of 18S and 28S RNA and the sizes of mRAR-γ transcripts are shown on the right. Open and filled triangles indicate putative splicing intermediate transcripts (see text). Arrowheads in the mRAR-γ1 and 2 panels indicate non-specific hybridization with 28S RNA (lanes 11 and 12). The arrowhead in the mRAR-γ3 panel points to a remnant of RAR-γ2 hybridization, due to incomplete dehybridization of the blot. Exposure time was 12 hours (mRAR-γ1 and 2; actin) or 60 hours (mRAR-γ3 and 6). (b) Experimental design for mRAR-γ1 transcripts quantification. A fragment from nucleotide 528 (Fig. 9a) to the 3′ end of the B region was subcloned in Bluescript SK+ between the BamHI and Not1 sites, yielding BSK-mRAR-γ1(A/B) which was linearized with EcoR1. An antisense riboprobe (192 nucleotides-long) was synthetized from the T3 promoter (T3). mRAR-γ1 and transcripts corresponding to the other mRAR-γ isoforms [mRAR-γ(Σ-1)] yielded 137 nucleotide-long and 83 nucleotide-long hybrids, respectively, following RNase digestion [Current Protocols in Molecular Biology. (eds Ausubel, F. M., Brent, R., Kingston, R. E., Moore, D. D., Seidman, J. G., Smith, J. A. & Struhl, K.) Greene Publishing Associates and Wiley-Interscience, John Willey & Sons, NY (1987)]. (c) Experimental design for mRAR-γ2 transcript quantification. A fragment from nucleotide 399 (Fig. 9b) to the 3′ end of the B region was subcloned into the BamHI site of Bluescript SK+, yielding BSK-mRAR-γ2(A/B) which was linearized with EcoR1. An antisense riboprobe (196 nucleotides-long) was synthetized with T3 polymerase. mRAR-γ2 and transcripts corresponding to the other mRAR-γ isoforms [mRAR-γ(Σ-2)] yielded 126 nucleotide-long and 83 nucleotide-long hybrids, respectively. (d) and (e). RNase protection assays for mRAR-γ1 and mRAR-γ2 transcripts were performed with mRAR-γ1 (panel d) or mRAR-γ2 (panel e) specific probes (see above and lane 1) using either 1 μg of polyA+ RNA from various sources as indicated [symbols as in panel (a)], 10 μg of total RNA from untreated (-) or RA-treated (+) ES cells, or 5 μg of tRNA (lane 13). Exposure time was 3 hours (lane 1), 48 hours (lanes 2-7) and 120 hours (lanes 8-13). Relative values given at the bottom in percentage have been calculated from the densitometric scanning of the bands corresponding to the protected fragments and indicate the amount of mRAR-γ1 (d) or mRAR-γ2 (e) transcripts relative to total RAR-γ transcripts. Scanning values have been standarized with respect to the number of labeled C's present in each protected fragment. Error is estimated to be within 5-10%.

## DEPOSITS

The DNA sequence encoding mRAR-α, mRAR-β, mRAR-γ1, mRAR-γ2, and hRAR-γ were deposited with the Collection Nationale de Culture De Microorganismes, 25 Rue Du Docteur Rout, 75724 Paris Cedex 15, France under the Budapest Treaty prior to June 29, 1990.

## Claims

1. A cDNA coding for the expression of a mouse retinoic acid receptor γ selected from the group consisting of mRAR-γ1, mRAR-γ2, mRAR-γ3, mRAR-γ4, mRAR-γ5, mRAR-γ6 and mRAR-γ7.

2. A mouse retinoic acid receptor γ selected from the group consisting of mRAR-γ1, mRAR-γ2, mRAR-γ3, mRAR-γ4, mRAR-γ5, mRAR-γ6 and mRAR-γ7.

3. A recombinant expression vector containing a DNA sequence encoding a mouse retinoic acid receptor γ selected from the group consisting of mRAR-γ1, mRAR-γ2, mRAR-γ3, mRAR-γ4, mRAR-γ5, mRAR-γ6 and mRAR-γ7, wherein the vector is capable of expressing the mouse retinoic acid receptor in a transformed microorganism, cell culture or transgenic animal.

4. A microorganism, cell culture or transgenic animal capable of expressing mouse retinoic acid receptor, obtained by transformation with the expression vector of claim 3.

5. A host cell transformed or transfected with the expression vector according to Claim 3.

6. A method of producing a mouse retinoic acid receptor which comprises culturing a microorganism or cell culture transformed with the vector of claim 3 or other expression vectors be they viral or plasmidic and recovering the retinoic acid receptor.

7. A cDNA coding for the expression of a human retinoic acid receptor γ protein.

8. The cDNA of claim 7 wherein the base sequence of the cDNA is as described in Figure 5.

9. A cDNA having the same or substantial sequence homology with cDNA A, B, C, D or E described in Figure 6.

10. A human retinoic acid receptor γ protein made by recombinant means.

11. A human retinoic acid receptor γ protein having an amino acid sequence as described in Figure 5.

12. A protein encoded by the cDNA of claim 8.

13. A protein encoded by cDNA, A, B, C, D, or E as described in Figure 6.

14. The protein recited in claim 10 wherein the protein binds specifically to retinoic acid found in skin.

15. A cDNA coding for the expression of a protein which has binding and transcription-activating properties characteristic of a skin-specific retinoic acid receptor γ.

16. A recombinant expression vector containing a DNA sequence encoding a human retinoic acid receptor γ protein, wherein the vector is capable of expressing retinoic acid receptor γ protein in a transformed microorganism or cell culture.

17. The recombinant expression vector of claim 16 wherein the recombinant DNA encoding the retinoic acid receptor protein has a DNA sequence as described in Figure 5.

18. The recombinant expression vector of claim 16 wherein the vector comprises a EcoRI-EcoRI cDNA fragment inserted into a eukaryotic expression vector pSG5 under the control of a SV40 early promoter.

19. A microorganism culture or cell culture capable of expressing human retinoic acid γ receptor, obtained by transforming the culture with the expression vector of claim 16.

20. The culture of claim 19 wherein the cells are mammalian.

21. The cell culture of claim 20 wherein the cells are HeLa.

22. The culture of claim 19 wherein the expressed retinoic acid receptor has the amino acid sequence set forth in Figure 5.

23. A method of producing recombinant human retinoic acid receptor γ which comprises culturing the cells of claim 19 and recovering the retinoic acid receptor.

24. A method for assaying a fluid for the presence of an agonist or antagonist to a retinoic acid receptor RAR and serotypic variants or fragments thereof, wherein the method comprises:

(A) providing an aqueous solution. containing a known concentration of a RAR protein and serotypic variants or fragments thereof;

(B) incubating the RAR protein with the fluid suspected of containing the agonist or antagonist under conditions sufficient to bind the receptor to the agonist or antagonist; and

(C) determining whether there is a change in concentration of the RAR protein in the aqueous solution.

25. The Method as claimed in claim 24, wherein the RAR protein and the agonist or antagonist form a complex.

26. The Method as claimed in claim 25, wherein a crosslinking agent is present in an amount sufficient to inhibit dissociation of the RAR protein and the agonist or antagonist.

27. The Method as claimed in claim 24, in which the RAR protein is free from human, blood-derived protein.

28. The Method as claimed in claim 24, wherein the RAR protein comprises a peptide fragment having the amino acid sequence of a mRARγ selected from the group consisting of mRAR-γ1, mRAR-γ2, mRAR-γ3, mRAR-γ4, mRAR-γ5, mRAR-γ6 and mRAR-γ7.

29. The Method as claimed in claim 24, wherein the RAR protein comprises a peptide fragment having the amino acid sequence of a hRARγ.

a.

1 Met Ala Ser Asn Ser Ser Ser Cys Pro Thr Pro Gly Gly Gly His Leu Asn Gly Tyr Pro A
21 Val Pro Pro Tyr Ala Phe Phe Phe Pro Pro Met Leu Gly Gly Leu Ser Pro Pro Gly Ala
41 Leu Thr Ser Leu Gln His Gln Leu Pro Val Ser Gly Tyr Ser Thr Pro Ser Pro Ala Thr
Thr
B
61 Ile Glu Thr Gln Ser Ser Ser Ser Glu Glu Ile Val Pro Ser Pro Pro Ser Pro Pro Pro
81 Leu Pro Arg Ile Tyr Lys Pro Cys Phe Val Cys Gln Asp Lys Ser Ser Gly Tyr His Tyr
101 Gly Val Ser Ala Cys Glu Gly Cys Lys Gly Phe Phe Arg Arg Ser Ile Gln Lys Asn Met C
121 Val Tyr Thr Cys His Arg Asp Lys Asn Cys Ile Ile Asn Lys Val Thr Arg Asn Arg Cys
141 Gln Tyr Cys Arg Leu Gln Lys Cys Phe Asp Val Gly Met Ser Lys Glu Ser Val Arg Asn
Glu
D
161 Asp Arg Asn Lys Lys Lys Lys Glu Ala Pro Lys Pro Glu Cys Ser Glu Ser Tyr Thr Leu
Val
181 Thr Pro Glu Val Gly Glu Leu Ile Glu Lys Val Arg Lys Ala His Gln Glu Thr Phe Pro
201 Ala Leu Cys Gln Leu Gly Lys Tyr Thr Thr Asn Asn Ser Ser Glu Gln Arg Val Ser Leu
221 Asp Ile Asp Leu Trp Asp Lys Phe Ser Glu Leu Ser Thr Lys Cys Ile Ile Lys Thr Val
241 Glu Phe Ala Lys Gln Leu Pro Gly Phe Thr Thr Leu Thr Ile Ala Asp Gln Ile Thr Leu
261 Leu Lys Ala Ala Cys Leu Asp Ile Leu Ile Leu Arg Ile Cys Thr Arg Tyr Thr Pro Glu
281 Gln Asp Thr Met Thr Phe Ser Asp Gly Leu Thr Leu Asn Arg Thr Gln Met His Asn Ala E
301 Gly Phe Gly Pro Leu Thr Asp Leu Val Phe Ala Phe Ala Asn Gln Leu Leu Pro Leu Glu
321 Met Asp Asp Ala Glu Thr Gly Leu Leu Ser Ala Ile Cys Leu Ile Cys Gly Asp Arg Gln
341 Asp Leu Glu Gln Pro Asp Lys Val Asp Met Leu Gln Glu Pro Leu Leu Glu Ala Leu Lys
Arg
361 Val Tyr Val Arg Lys Arg Arg Pro Ser Arg Pro His Met Phe Pro Lys Met Leu Met Lys
381 Ile Thr Asp Leu Arg Ser Ile Ser Ala Lys Gly Ala Glu Arg Val Ile Thr Leu Lys Met
401 Glu Ile Pro Gly Ser Met Pro Pro Leu Ile Gln Glu Met Leu Glu Asn Ser Glu Gly Leu
421 Asp Thr Leu Ser Gly Gln Ser Gly Gly Gly Thr Arg Asp Gly Gly Gly Leu Ala Pro Pro F
Pro Gly
441 Pro Gly Ser Cys Ser Pro Ser Leu Ser Pro Ser Ser His Arg Ser Ser Pro Ala Thr Gln
Asn His
461 Ser Pro End

b.

1 Met Phe Asp Cys Met Asp Val Leu Ser Val Ser Pro Gly Gln Ile Leu Asp Phe Tyr Thr A
21 Ala Ser Pro Ser Ser Cys Met Leu Gln Glu Lys Ala Leu Lys Ala Cys Leu Ser Gly Phe
Phe Leu
41 Thr Gln Ala Glu Trp Gln His Arg His Thr Ala Gln Ser Ile Glu Thr Gln Ser Thr Ser
Thr
B
61 Ser Glu Glu Leu Val Pro Ser Pro Pro Ser Pro Leu Pro Pro Pro Arg Val Tyr Lys Pro
81 Cys Phe Val Cys Gln Asp Lys Ser Ser Gly Tyr His Tyr Gly Val Ser Ala Cys Glu Gly
101 Cys Lys Gly Phe Phe Arg Arg Ser Ile Gln Lys Asn Met Ile Tyr Thr Cys His Arg Asp C
121 Lys Asn Cys Val Ile Asn Lys Val Thr Arg Asn Arg Cys Gln Tyr Cys Arg Leu Gln Lys
141 Cys Phe Glu Val Gly Met Ser Lys Glu Ser Val Arg Asn Asp Arg Asn Lys Lys Lys Lys
161 Glu Pro Ser Lys Gln Glu Cys Thr Glu Ser Tyr Glu Met Thr Ala Glu Leu Asp Asp Leu D
Thr
181 Thr Glu Lys Ile Arg Lys Ala His Gln Glu Thr Phe Pro Ser Leu Cys Gln Leu Gly Lys
201 Tyr Thr Thr Asn Ser Ser Ala Asp His Arg Val Arg Leu Asp Leu Gly Leu Trp Asp Lys
221 Phe Ser Glu Leu Ala Thr Lys Cys Ile Ile Lys Ile Val Glu Phe Ala Lys Arg Leu Pro
241 Gly Phe Thr Gly Leu Thr Ile Ala Asp Gln Ile Thr Leu Leu Lys Ala Ala Cys Leu Asp
261 Ile Leu Ile Leu Arg Ile Cys Thr Arg Tyr Thr Pro Glu Gln Asp Thr Met Thr Phe Ser E
281 Asp Gly Leu Thr Leu Asn Arg Thr Gln Met His Asn Ala Gly Phe Gly Pro Leu Thr Asp
301 Leu Val Phe Thr Phe Ala Asn Gln Leu Leu Pro Leu Glu Met Asp Asp Thr Glu Thr Gly
321 Leu Leu Ser Ala Ile Cys Leu Ile Cys Gly Asp Arg Gln Asp Leu Glu Glu Pro Thr Lys
341 Val Asp Lys Leu Gln Glu Pro Leu Leu Glu Ala Leu Lys Ile Tyr Ile Arg Lys Arg Arg
361 Pro Ser Lys Pro His Met Phe Pro Lys Ile Leu Met Lys Ile Thr Asp Leu Arg Ser Ile
381 Ser Ala Lys Gly Ala Glu Arg Val Ile Thr Leu Lys Met Glu Ile Pro Gly Ser Met Pro
401 Pro Leu Ile Gln Glu Met Leu Glu Asn Ser Glu Gly His Glu Pro Leu Thr Pro Ser Ser
Met
421 Ser Gly Asn Ile Ala Glu His Ser Pro Ser Val Ser Pro Ser Ser Val Glu Asn Ser Gly F
Thr Ile
441 Val Ser Gln Ser Pro Leu Leu Gln End
Val

FIG. 1-1

C.

```
1    AATTCCCCCCCCCCCCCCCCCACCGCGGTGCTCCATCCCCGAGACCGCCCGACGCCGGGACCTCGGGGCTCTGCGGCC
79   TTCCTTCCCCCGCCCTCCCCTCCAGCAGTTTCCACCAGGTCCCTCACCTCAGCCTGGCCCAGTATGTAGGAGGGACTCT
158  CTGCAGAGGCCAGAGGGATCCTTGGAAACCCACTGGACAGACCAGGCAGGGTGGGCACGGAGCCTCCCAGGCCAGGGCA
237  GTGGGCATGGGCGGGGGCTGTAGCTGAAGACCACCCCCGCCTGCTGCAGAGTCCAAGGGATTCCCACGCCGCAGCTACC

316  ATG GCC ACC AAT AAG GAG AGA CTC TTT GCG CCC GGT GCC CTG GGG CCT GGA TCT GGT TAC
1    Met Ala Thr Asn Lys Glu Arg Leu Phe Ala Pro Gly Ala Leu Gly Pro Gly Ser Gly Tyr

376  CCA GGA GCA GGC TTC CCA TTC GCC TTC CCA GGT GCA CTC AGA GGG TCG CCA CCA TTT GAG   A
21   Pro Gly Ala Gly Phe Pro Phe Ala Phe Pro Gly Ala Leu Arg Gly Ser Pro Pro Phe Glu

436  ATG CTG AGC CCT AGC TTC CGG GGC CTG GGC CAG CCT GAC CTC CCC AAG GAG ATG GCT TCT
41   Met Leu Ser Pro Ser Phe Arg Gly Leu Gly Gln Pro Asp Leu Pro Lys Glu Met Ala Ser

496  CTC TCG GTG GAG ACA CAG AGC ACC AGC TCG GAG GAG ATG GTA CCC AGC TCT CCC TCA CCC   B
61   Leu Ser Val Glu Thr Gln Ser Thr Ser Ser Glu Glu Met Val Pro Ser Ser Pro Ser Pro

556  CCA CCA CCT CCT CGG GTC TAT AAG CCA TGC TTT GTA TGC AAT GAC AAG TCT TCT GGC TAC
81   Pro Pro Pro Pro Arg Val Tyr Lys Pro Cys Phe Val Cys Asn Asp Lys Ser Ser Gly Tyr

616  CAC TAT GGG GTC AGC TCC TGT GAA GGC TGC AAG GGC TTC TTC AGA CGC AGC ATT CAG AAA   C
101  His Tyr Gly Val Ser Ser Cys Glu Gly Cys Lys Gly Phe Phe Arg Arg Ser Ile Gln Lys

676  AAC ATG GTG TAT ACA TGT CAC CGT GAC AAA AAC TGT ATC ATC AAC AAG GTC ACC AGA AAT
121  Asn Met Val Tyr Thr Cys His Arg Asp Lys Asn Cys Ile Ile Asn Lys Val Thr Arg Asn

736  CGA TGC CAG TAC TGC AGG CTA CAA AAG TGT TTC GAA GTG GGC ATG TCC AAG GAA GCT GTA
141  Arg Cys Gln Tyr Cys Arg Leu Gln Lys Cys Phe Glu Val Gly Met Ser Lys Glu Ala Val

796  AGG AAC GAT CGA AAC AAG AAG AAA AAG GAG GTA AAA GAG GAG GGC TCG CCC GAC AGC TAT   D
161  Arg Asn Asp Arg Asn Lys Lys Lys Lys Glu Val Lys Glu Glu Gly Ser Pro Asp Ser Tyr

856  GAA CTG AGT CCA CAG TTA GAG GAA CTC ATC ACC AAG GTC AGC AAA GCC CAC CAG GAG ACT
181  Glu Leu Ser Pro Gln Leu Glu Glu Leu Ile Thr Lys Val Ser Lys Ala His Gln Glu Thr

916  TTT CCC TCA CTC TGC CAG CTG GGC AAG TAC ACC ACG AAC TCC AGT GCA GAT CAC CGG GTG
201  Phe Pro Ser Leu Cys Gln Leu Gly Lys Tyr Thr Thr Asn Ser Ser Ala Asp His Arg Val

976  CAG CTG GAC CTG GGG CTG TGG GAC AAG TTC AGC GAG CTG GCC ACC AAA TGC ATC ATC AAG
221  Gln Leu Asp Leu Gly Leu Trp Asp Lys Phe Ser Glu Leu Ala Thr Lys Cys Ile Ile Lys

1036 ATT GTG GAG TTT GCG AAG CGG CTG CCT GGT TTT ACA GGG CTC AGC ATT GCC GAC CAG ATC
241  Ile Val Glu Phe Ala Lys Arg Leu Pro Gly Phe Thr Gly Leu Ser Ile Ala Asp Gln Ile

1096 ACG CTG CTC AAG GCT GCT TGT CTG GAC ATC CTA ATG CTG CGG ATC TGT ACA AGG TAT ACC
261  Thr Leu Leu Lys Ala Ala Cys Leu Asp Ile Leu Met Leu Arg Ile Cys Thr Arg Tyr Thr

1156 CCA GAG CAG GAC ACT ATG ACA TTC TCG GAT GGG CTG ACC CTG AAC CGA ACC CAG ATG CAC
281  Pro Glu Gln Asp Thr Met Thr Phe Ser Asp Gly Leu Thr Leu Asn Arg Thr Gln Met His   E

1216 AAT GCT GGC TTT GGG CCC CTT ACA GAC CTC GTC TTT GCC TTT GCC GGG CAG CTG CTG CCC
301  Asn Ala Gly Phe Gly Pro Leu Thr Asp Leu Val Phe Ala Phe Ala Gly Gln Leu Leu Pro

1276 CTG GAG ATG GAT GAC ACC GAG ACT GGG CTA CTT AGT GCT ATC TGC CTC ATC TGT GGA GAC
321  Leu Glu Met Asp Asp Thr Glu Thr Gly Leu Leu Ser Ala Ile Cys Leu Ile Cys Gly Asp

1336 CGA ATG GAC CTG GAA GAG CCC GAG AAG GTG GAC AAG CTG CAG GAG CCC CTG CTG GAA GCC
341  Arg Met Asp Leu Glu Glu Pro Glu Lys Val Asp Lys Leu Gln Glu Pro Leu Leu Glu Ala

1396 CTG AGG CTC TAT GCC CGG CGA CGG AGA CCC AGC CAA CCC TAC ATG TTC CCA AGG ATG CTG
361  Leu Arg Leu Tyr Ala Arg Arg Arg Arg Pro Ser Gln Pro Tyr Met Phe Pro Arg Met Leu

1456 ATG AAA ATC ACC GAC CTC CGG GGC ATC AGC ACT AAG GGA GCA GAA AGG GCT ATA ACC CTG
381  Met Lys Ile Thr Asp Leu Arg Gly Ile Ser Thr Lys Gly Ala Glu Arg Ala Ile Thr Leu

1516 AAG ATG GAG ATT CCA GGC CCG ATG CCA CCC CTG ATC CGA GAG ATG CTG GAG AAC CCG GAG
401  Lys Met Glu Ile Pro Gly Pro Met Pro Pro Leu Ile Arg Glu Met Leu Glu Asn Pro Glu

1576 ATG TTT GAG GAC GAC TCC TCG AAG-CCT GGC CCC CAC CCC AAG GCT TCC AGT GAG GAC GAA
421  Met Phe Glu Asp Asp Ser Ser Lys Pro Gly Pro His Pro Lys Ala Ser Ser Glu Asp Glu   F

1636 GCT CCA GGG GGC CAG GGC AAA AGG GGC CAA AGT CCC CAA CCT GAC CAG GGG CCC TGACCTA
441  Ala Pro Gly Gly Gln Gly Lys Arg Gly Gln Ser Pro Gln Pro Asp Gln Gly Pro

1697 CCCCGTTGTGGGGTTGGCCCCAGGCAGCAGACTGACCATTTCCCAGATACCGCCAGTGACTGGGGGAGGACCTGCCCC
1776 GCCCACTCTCCACCCTTTTAATGAGCTCGTTATTTTGCCAAAGTTTCTAGGGGTGCCTGTGTTCATCCCTGTCCTGTTC
1855 TAACTGGCTCCCTCTACAGTCCCGCGGACTGCTGCACACCTGGGGGGGGGGAATTC
```

FIG. 1-2

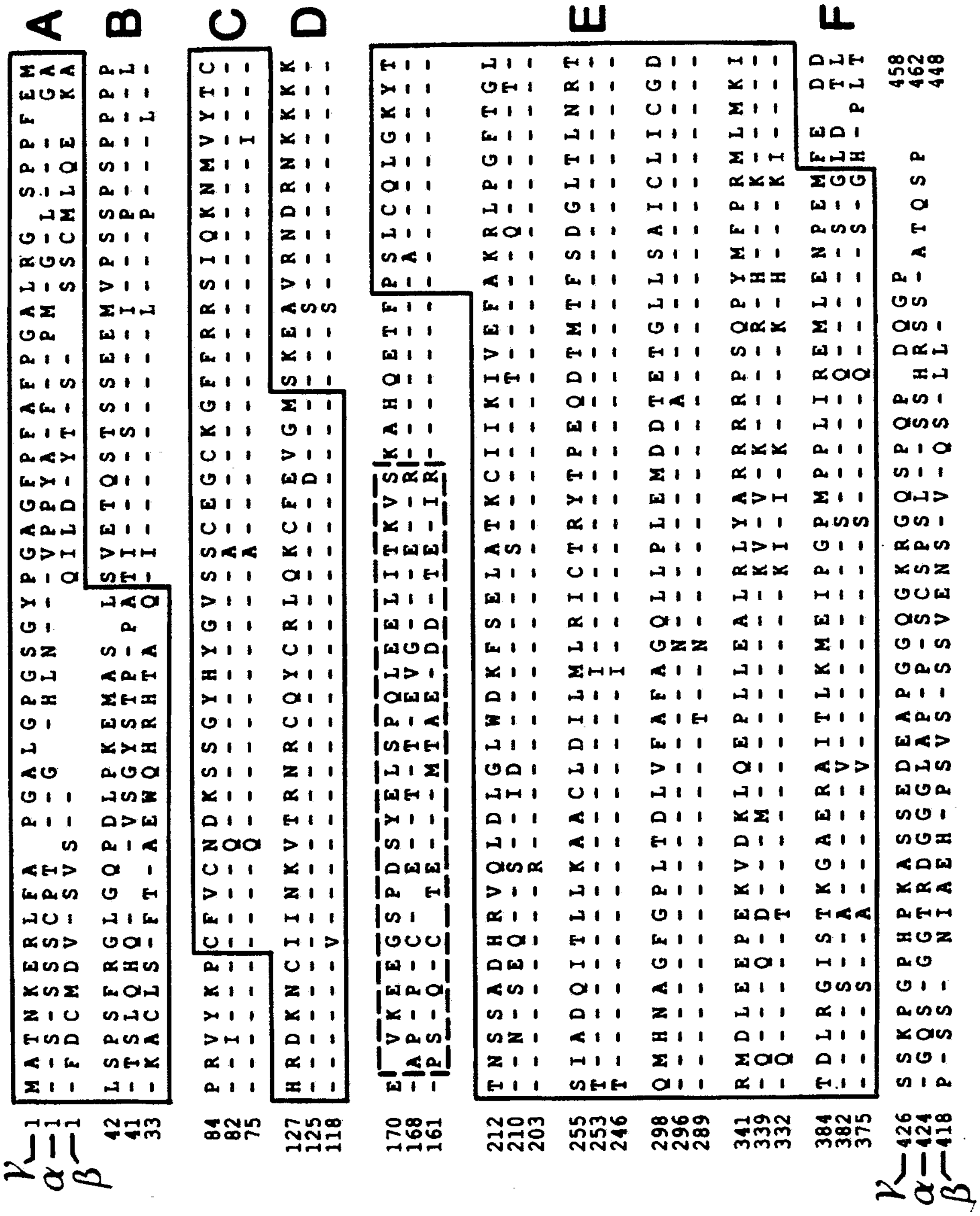

FIG. 2

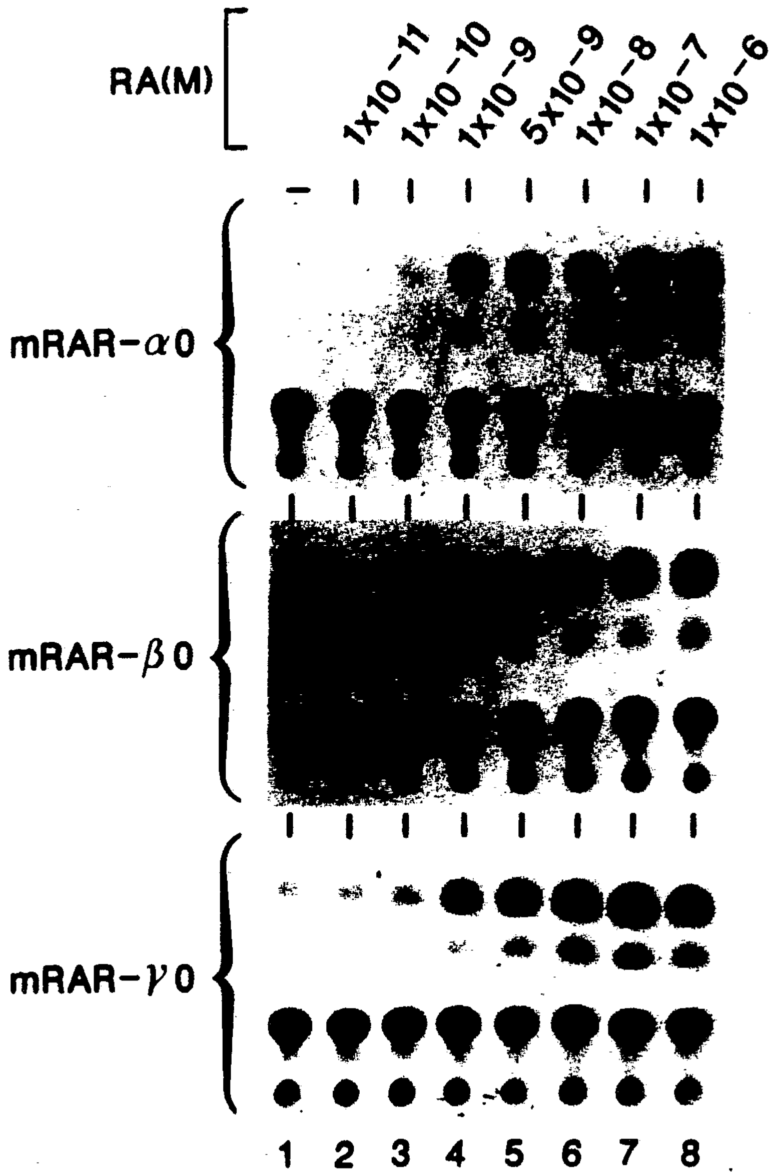
RA(M)
-
1x10 -11
1x10 -10
1x10 -9
5x10 -9
1x10 -8
1x10 -7
1x10 -6
mRAR-α0
mRAR-β0
mRAR-γ0
1 2 3 4 5 6 7 8

FIG. 3

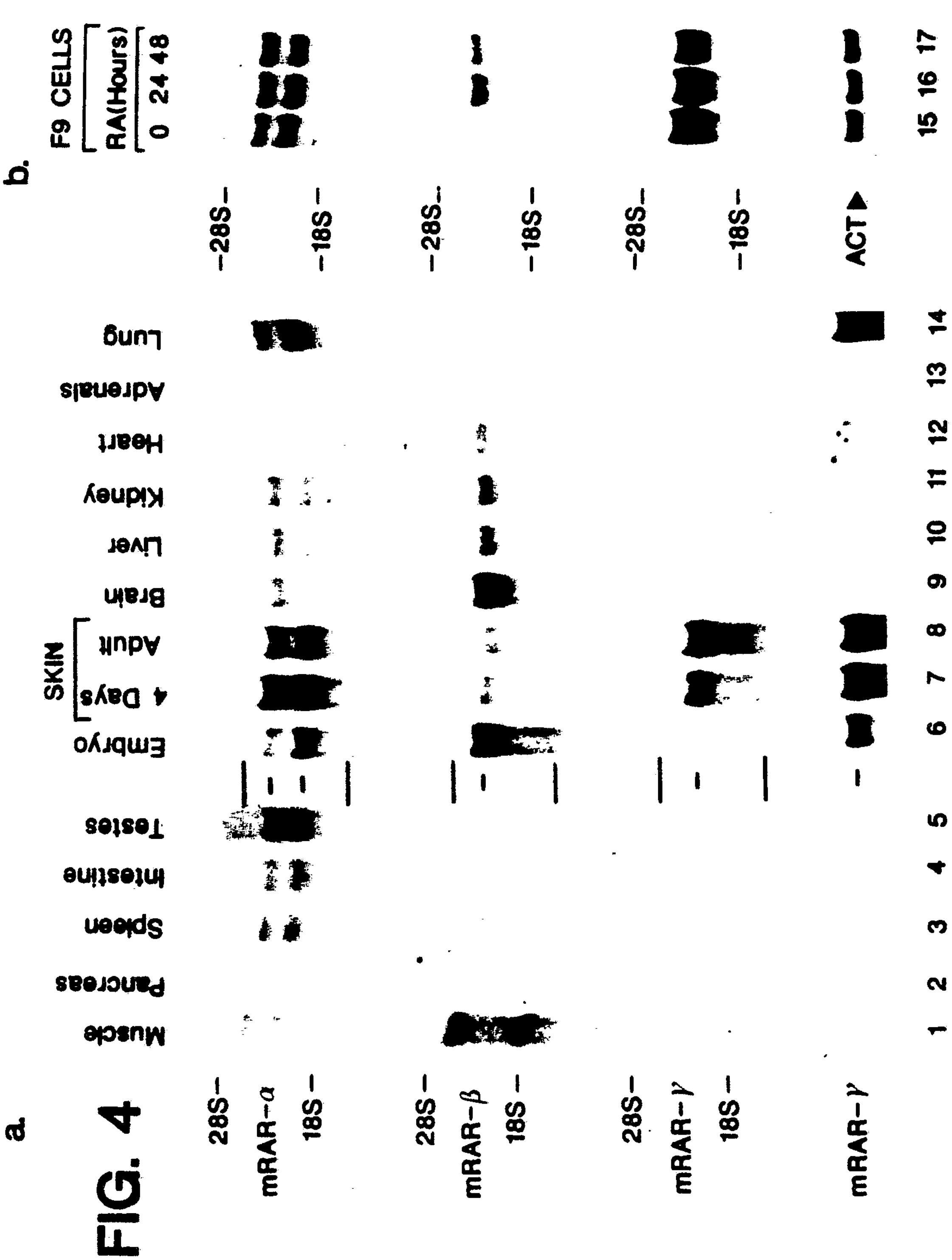
a.
b.
Muscle
Pancreas
Spleen
Intestine
Testes
Embryo
SKIN
4 Days
Adult
Brain
Liver
Kidney
Heart
Adrenals
Lung
F9 CELLS
RA(Hours)
0 24 48
28S
mRAR-α
18S
28S
mRAR-β
18S
28S
mRAR-γ
18S
mRAR-γ
ACT
1 2 3 4 5 6 7 8 9 10 11 12 13 14
15 16 17

FIG. 4

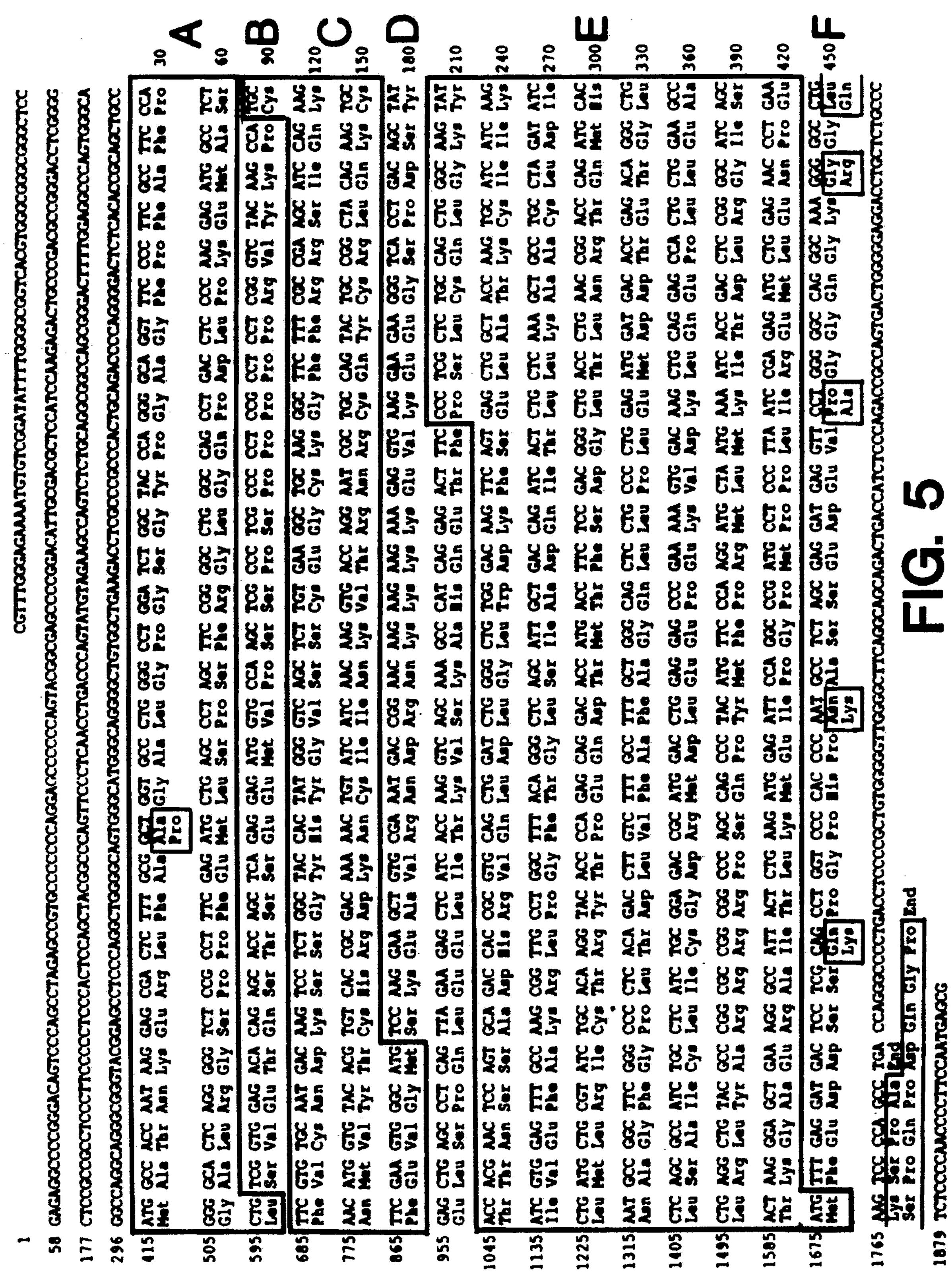


FIG. 5

hRAR-γ

```
  1 CGTTTGGGAGAAAATGTGTCGGATATTTTGGGGCGGTCACGTGGGCGGGCGGCTCCGAGAGGCCCCGGGACAGTCCCAGCCTAGAGCCGTGCCCCCCCAGGAGCCCCCCAGTA ——— A
  1       GGGAGAAAATGTGTCGGATATTTTGGGGCGGTCACGTGGGCGGGCGGCTCCGAGAGGCCCCGGGACAGTCCCAGCCTAGAGCCGTGCCCCCCCAGGAGCCCCCCAGTA ——— B

115 CGGCGACCCCCGGACATTCGGACGCTCCATCCAAGCGACTGGCCGACGCCGGGACCTCGGGGCTCCGCCGCCTCCCTTCCCCCTCCCACTCCAGCTACGGCCCAGTTCCCTCA ——— A
110 CGGCGACCCCCGGACATTCGGACGCTCCATCCAAGCGACTGGCCGACGCCGGGACCTCGGGGCTCCGCCGCCTCCCTTCCCCCTCCCACTCCAGC.................... ——— B
  1 CGGCGAGCCCCGGACATTCGGACGCTCCATCCAAGAGACTGCCCGACGCCGGGACCTCGGGGCTCCGCCGCCTCCCTTCCCCCTCCCACTCCAGCTACGGCCCAGTTCCCTCA ——— C

228 ACCTGACCCAGTATGTAGAAGCCAGTCTCTGCAGGCGGCCAGCGG.................................................................... ——— A
    ................................................................................................................. ——— B
114 ACCTGACCCAGTATGTAGAAGCCAGTCTCTGCAGGCGGCCAGCGGCCACGACAGAACCAAGGCTCAGAAATGGAGGAATCTCTACCCCCTTCCTCTCTTCACTCAACTCCCA ——— C

273 ..................................................................................GACTTTTGGAGGCCCAGTGGGCAGGCCAGGCAGGGCG ——— A
    ................................................................................................................. ——— B
227 AGTCCCTTCTCTGAGGCAAAATAAGAGGAAGGCCAAGGACAACTCTCATTGGCTCACATGCAGCATCATTCCTTGGACTTTTGGAGGCCCAGTGGGCAGGCCAGGCAGGGCG ——— C

310 GGTACGGAGCCTCCCAGGCTGGGGCAGTGGGCATGGGCAGGGGCTGTGGCTGAAGACCTCGCCCGCCCACTGCAGACCCCAGGGGACTCTCACACCGCAGCTGCCATGGCCAC ——— A
    ................................................................................................................. ——— B
340 GGTACGGAGCCTCCCAGGCTGGGGCAGTGGGCATGGGCAGGGGCTGTGGCTGAAGACCTCGCCCGCCCACTGCAGACCCCAGGGGACTCTCACACCGCAGCTGCCATGGCCAC ——— C
  1                                                               CCCCCTGCCCATGCCCACTGCCCCAGGGGACTCTCACACCGCAGCTGCCATGGCCAC ——— D

423 CAATAAGGAGCGACTCTTTGCGGCTGGTGCCCTGGGGCCTGGATCTGGCTACCCAGGGGCAGGTTTCCCCTTCGCCTTCCCAGGGGCACTCAGGGGGTCTCCGCCTTTCGAGA ——— A
    ................................................................................................................. ——— B
453 CAATAAGGAGCGACTCTTTGCGGCTGGTGCCCTGGGGCCTGGATCTGGCTACCCAGGGGCAGGTTTCCCCTTCGCCTTCCCAGGGGCACTCAGGGGGTCTCCGCCTTTCGAGA ——— C
 58 CAATAAGGAGCGACTCTTTGCGGCTGGTGCCCTGGGGCCTGGATCTGGCTACCCAGGGGCAGGTTTCCCCTTCGCCTTCCCAGGGGCACTCAGGGGGTCTCCGCCTTTCGAGA ——— D

                                                                           ▼
536 TGCTGAGCCCTAGCTTCCGGGGCCTGGGCCAGCCTGACCTCCCCAAGGAGATGGCCTCTCTGTCGGTGGAGACACAGAGCACCAGCTCAGAGGAGATGGTGC-//-- 2000 ——— A
205 ...............................................................GGTGGAGACACAGAGCACCAGCTCAGAGGAGATGGTGC-//-- 1136 ——— B
566 TGCTGAGCCCTAGCTTCCGGGGCCTGGGCCAGCCTGACCTCCCCAAGGAGATGGCCTCTCTGTCGGTGGAGACACAGAGCACCAGCTCAGAGGAGATGGTGC-//-- 1597 ——— C
171 TGCTGAGCCCTAGCTTCCGGGGCCTGGGCCAGCCTGACCTCCCCAAGGAGATGGCCTCTCTGTCGGTGGAGACACAGAGCACCAGCTCAGAGGAGATGGTGC-//-- 1544 ——— D
  1                             CCTTTTGCCTGGCCGCAACCCGCCAGCCTGCAATCGGTGGAGACACAGAGCACCAGCTCAGAGGAGATGGTGC-//-- 2200 ——— E
```

FIG. 6

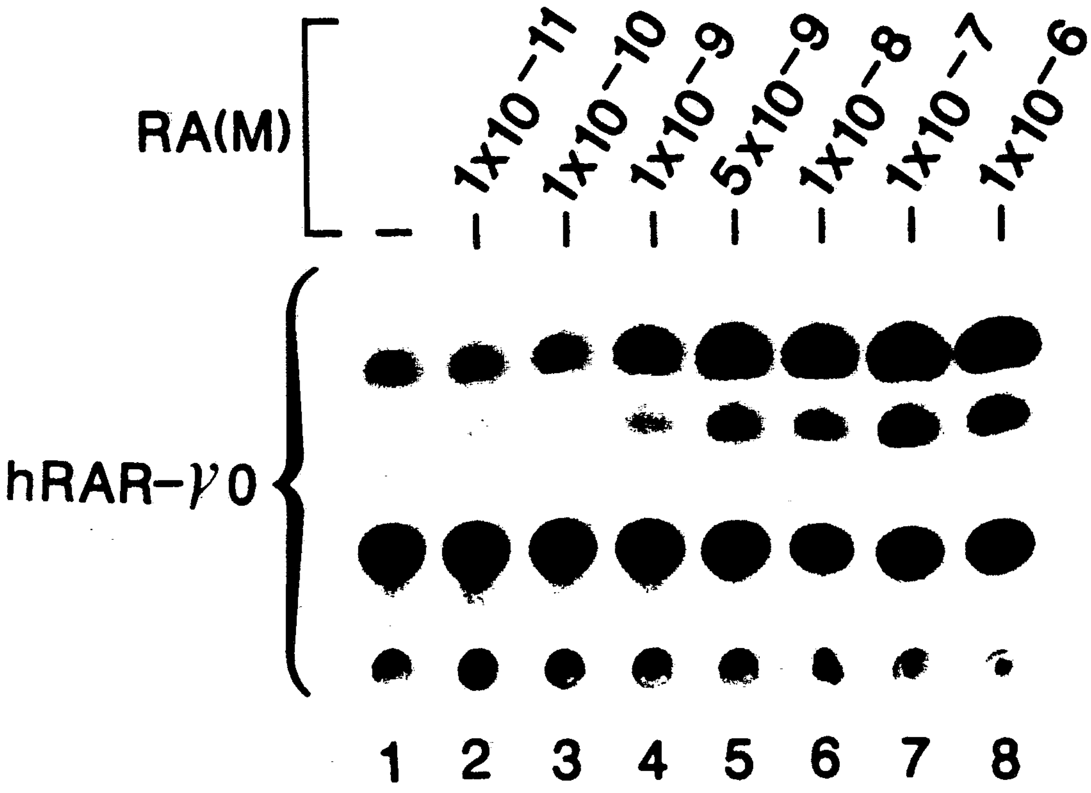
RA(M)
–
1x10⁻¹¹
1x10⁻¹⁰
1x10⁻⁹
5x10⁻⁹
1x10⁻⁸
1x10⁻⁷
1x10⁻⁶
hRAR-γ0
1 2 3 4 5 6 7 8

FIG. 7

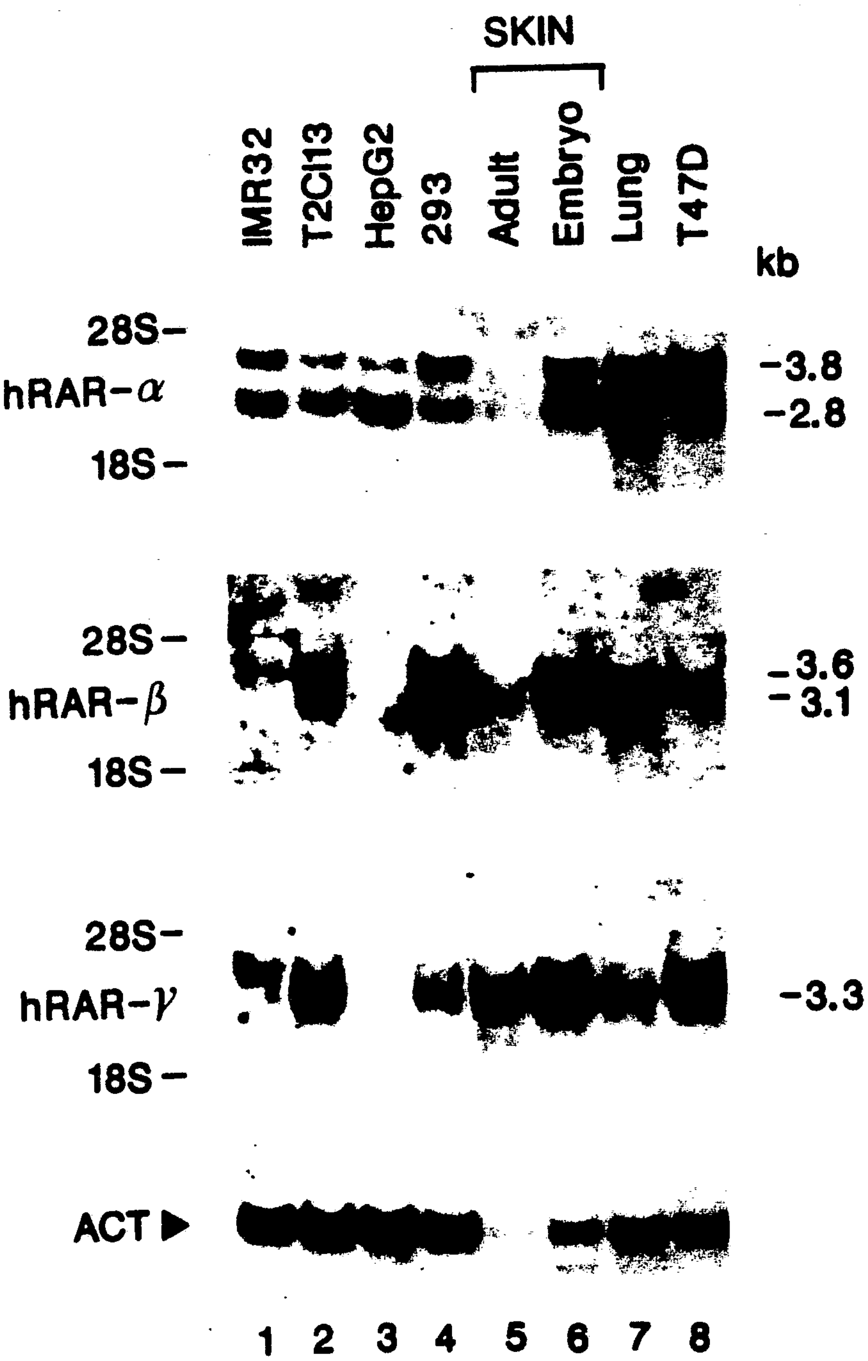
SKIN
IMR32
T2Cl13
HepG2
293
Adult
Embryo
Lung
T47D
kb
28S-
hRAR-α
18S-
-3.8
-2.8
28S-
hRAR-β
18S-
-3.6
-3.1
28S-
hRAR-γ
18S-
-3.3
ACT ▶
1 2 3 4 5 6 7 8

FIG. 8

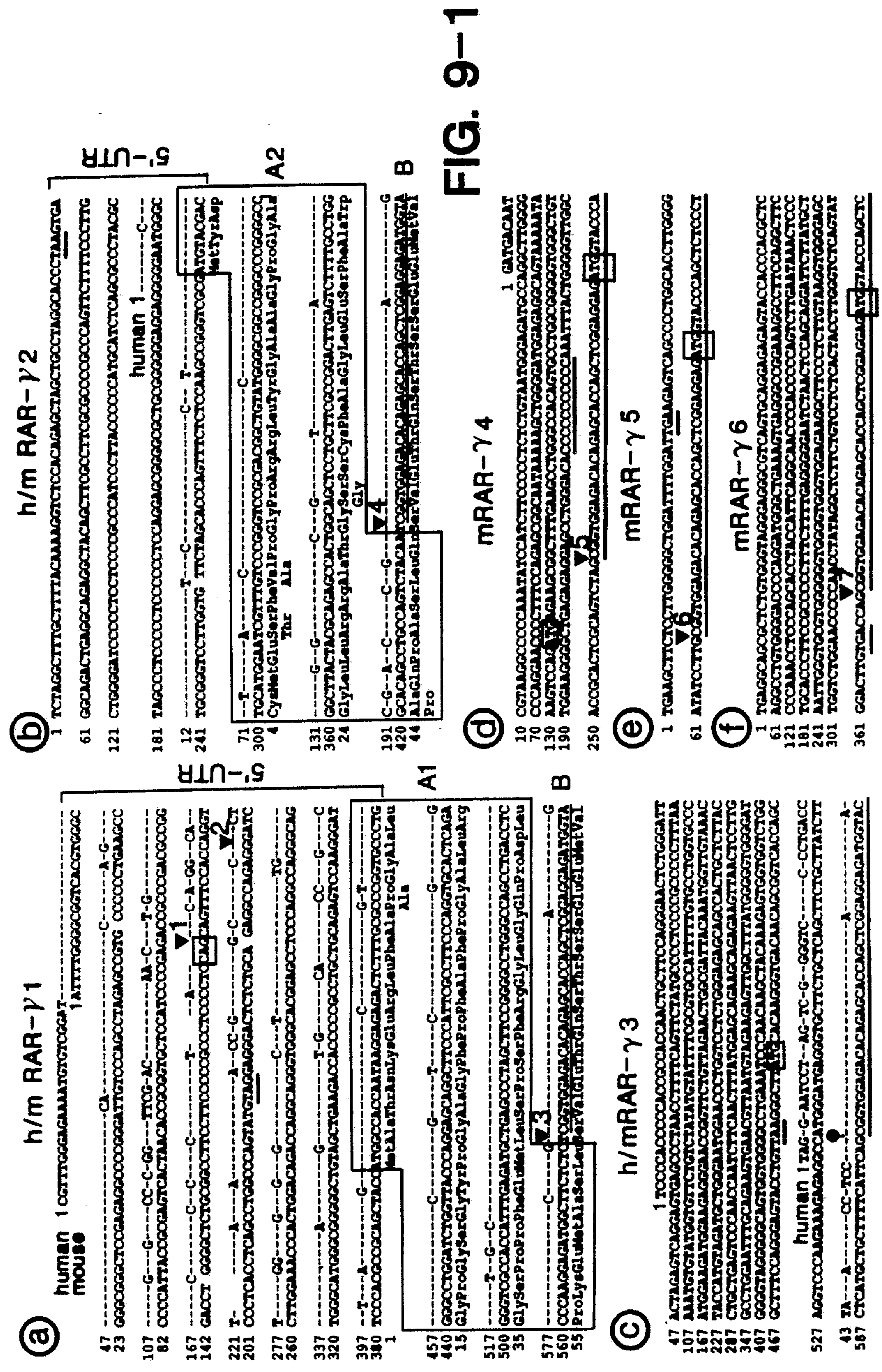

h/m RAR-γ1
human
mouse
5'-UTR
A1
B
h/mRAR-γ3
human
h/m RAR-γ2
human 1
5'-UTR
A2
B
mRAR-γ4
mRAR-γ5
mRAR-γ6


FIG. 9-1

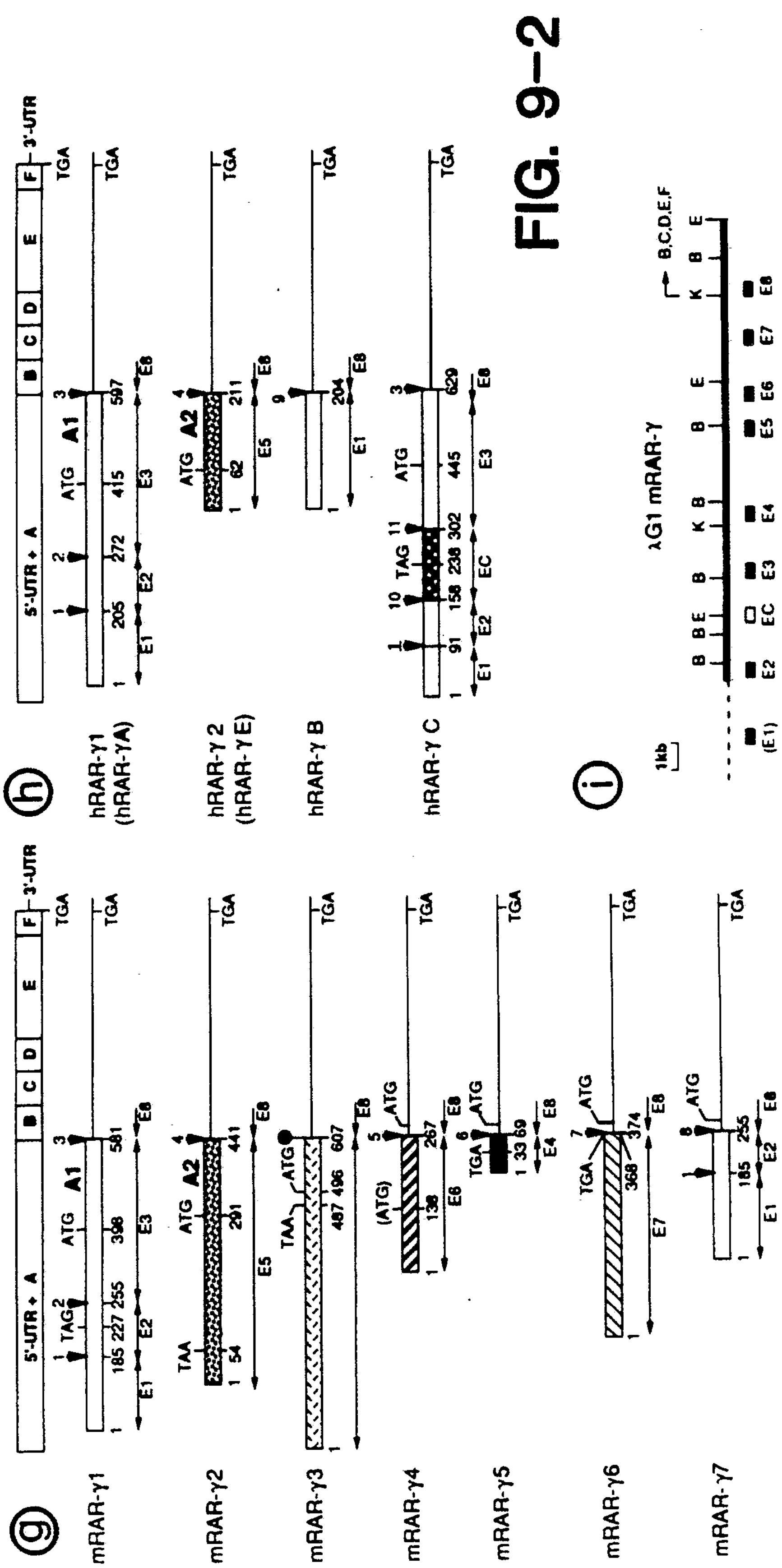
g
mRAR-γ1
mRAR-γ2
mRAR-γ3
mRAR-γ4
mRAR-γ5
mRAR-γ6
mRAR-γ7
5'-UTR + A
B C D E F
3'-UTR
A1
A2
ATG
TAG
TAA
TGA
(ATG)
E1 E2 E3 E4 E5 E6 E7 E8
h
hRAR-γ1 (hRAR-γA)
hRAR-γ2 (hRAR-γE)
hRAR-γB
hRAR-γC
EC
i
λG1 mRAR-γ
1kb
B,C,D,E,F
(E1)

FIG. 9–2

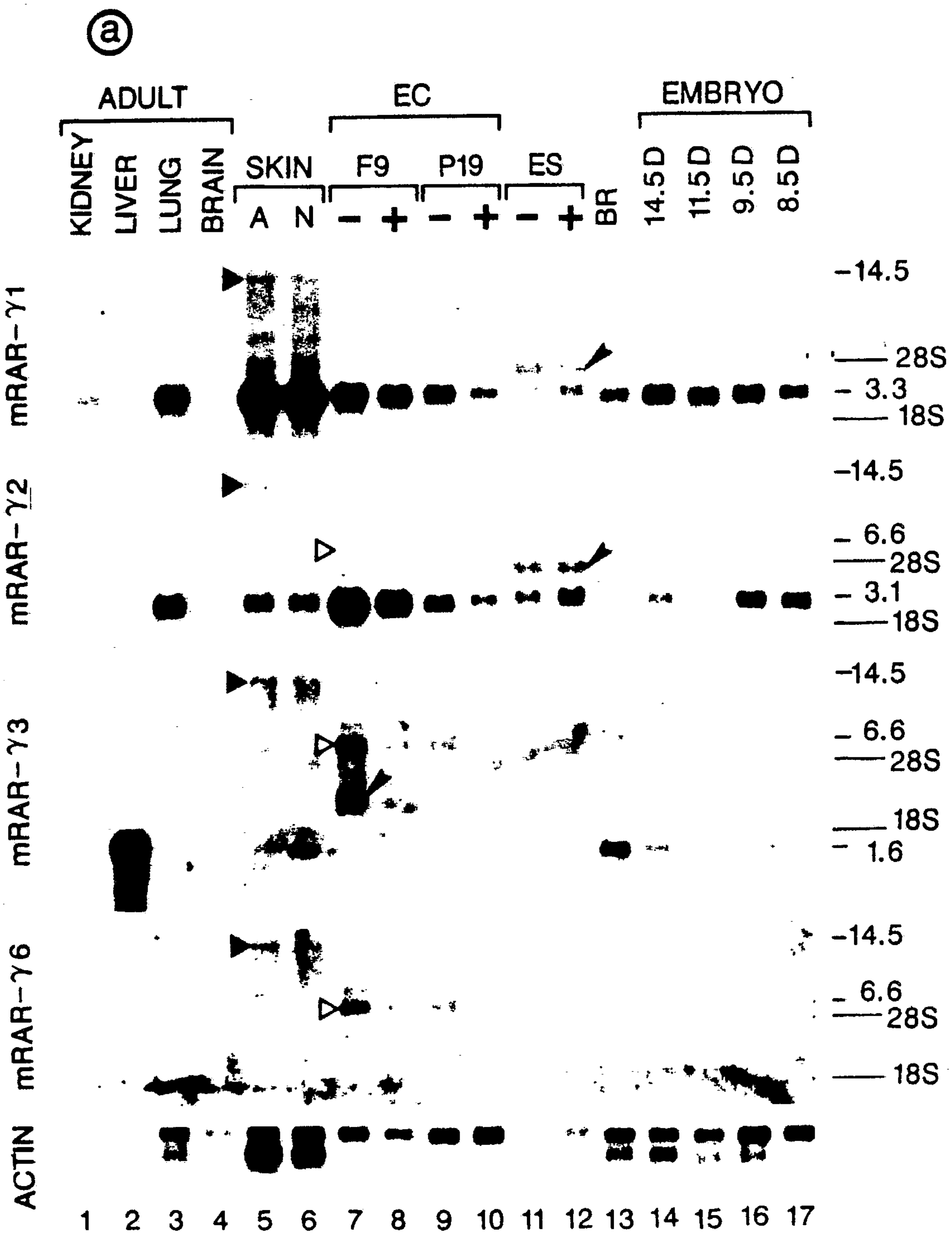
a
ADULT
EC
EMBRYO
KIDNEY
LIVER
LUNG
BRAIN
SKIN
A
N
F9
P19
ES
− + − + − +
BR
14.5 D
11.5 D
9.5 D
8.5 D
mRAR-γ1
mRAR-γ2
mRAR-γ3
mRAR-γ6
ACTIN
-14.5
28S
3.3
18S
-14.5
6.6
28S
3.1
18S
-14.5
6.6
28S
18S
1.6
-14.5
6.6
28S
18S
1 2 3 4 5 6 7 8 9 10 11 12 13 14 15 16 17

FIG. 10–1

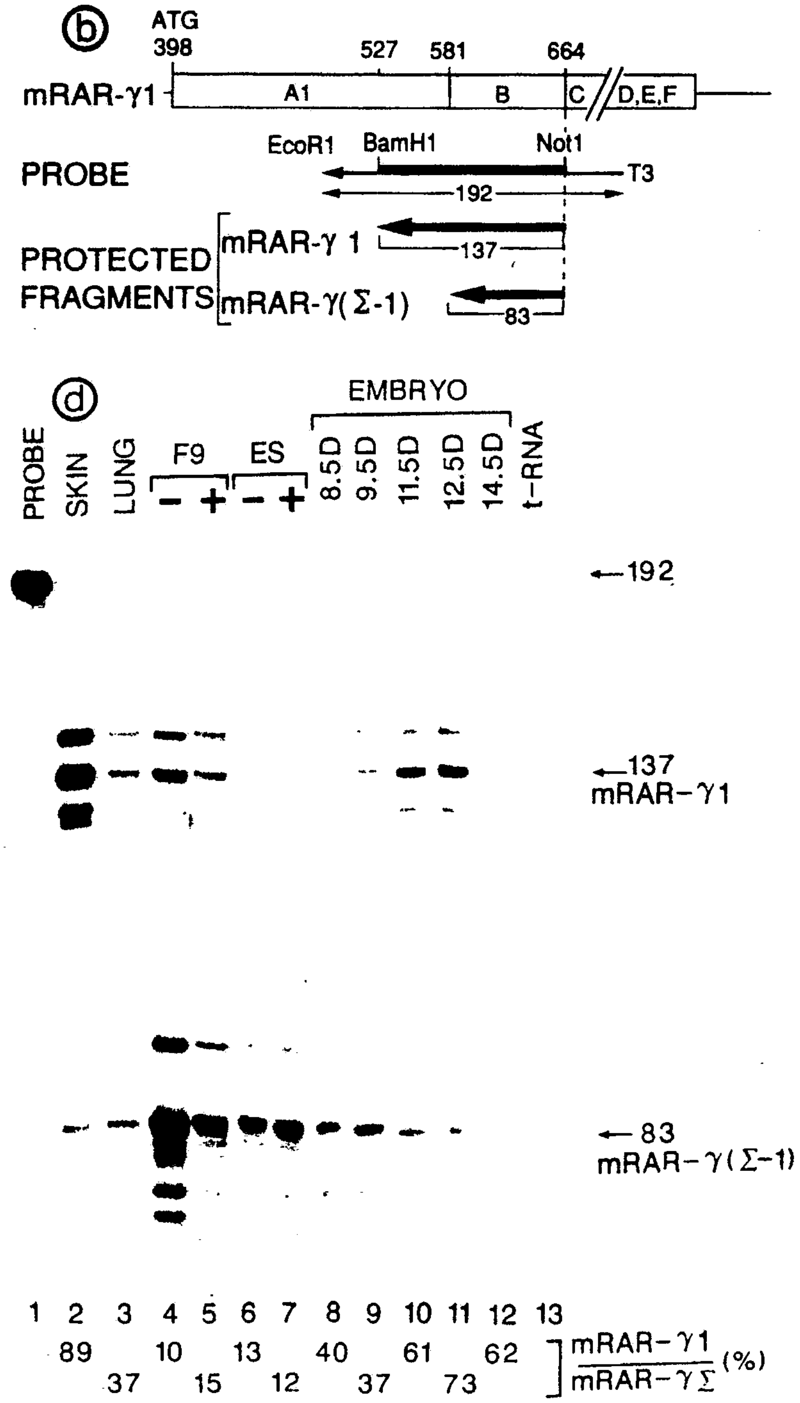
b
ATG
398
527
581
664
mRAR-γ1
A1
B
C
D,E,F
EcoR1
BamH1
Not1
PROBE
T3
192
PROTECTED
FRAGMENTS
mRAR-γ 1
137
mRAR-γ(Σ-1)
83
d
EMBRYO
PROBE
SKIN
LUNG
F9
ES
- + - +
8.5D
9.5D
11.5D
12.5D
14.5D
t-RNA
←192
←137
mRAR-γ1
← 83
mRAR-γ(Σ-1)
1 2 3 4 5 6 7 8 9 10 11 12 13
89 10 13 40 61 62
37 15 12 37 73
mRAR-γ1 / mRAR-γΣ (%)

FIG. 10-2

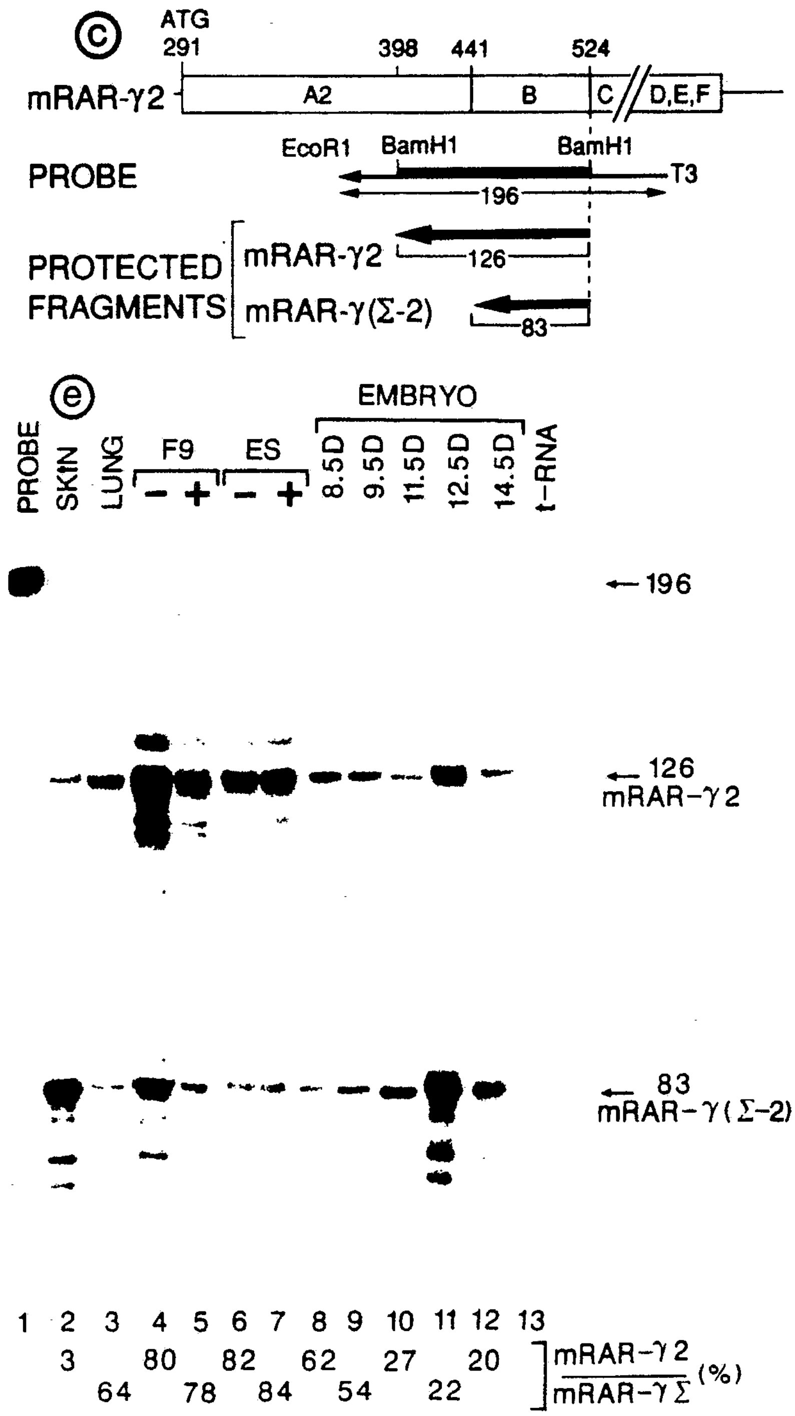
c
ATG
291
398
441
524
mRAR-γ2
A2
B
C
D,E,F
EcoR1
BamH1
BamH1
PROBE
T3
196
PROTECTED FRAGMENTS
mRAR-γ2
126
mRAR-γ(Σ-2)
83
e
EMBRYO
PROBE
SKIN
LUNG
F9
ES
- + - +
8.5D
9.5D
11.5D
12.5D
14.5D
t-RNA
196
126 mRAR-γ2
83 mRAR-γ(Σ-2)
1 2 3 4 5 6 7 8 9 10 11 12 13
3 80 82 62 27 20
64 78 84 54 22
mRAR-γ2 / mRAR-γΣ (%)

FIG. 10-3

Table 1

| I | II | III | IV | V | VI | VII | VIII |
|---|---|---|---|---|---|---|---|
| | hγ/mγ | hα/mα | hβ/mβ | hγ/hα | hγ/hβ | hα/hβ | hER/mER |
| A | 98 | 98 | 94 | 25 | <15 | <15 | 95 |
| B | 100 | 100 | 100 | 75 | 86 | 79 | 80 |
| C | 100 | 98 | 100 | 97 | 94 | 97 | 100 |
| D | 100 | 98 | 98 | 72 | 62 | 74 | 80 |
| E | 100 | 99 | 99 | 84 | 90 | 90 | 96 |
| F | 58 | 90 | 92 | 20 | <15 | 20 | 60 |